# EUROPEAN PATENT SPECIFICATION

(11) **EP 1 873 143 B1**
(45) Date of publication and mention of the grant of the patent: **15.04.2009**
(21) Application number: 06013261.0
(22) Date of filing: 27.06.2006
(51) Int. Cl.: C07C 309/17, H01L 21/027

(54) **A salt suitable for an acid generator and a chemically amplified resist composition containing the same**
Salz, welches für einen Sauergenerator geeignet ist, sowie chemisch verstärkte Resistzusammensetzung, die dieses enthält
Sel approprié pour un générateur d'acide et composition de réserve chimiquement amplifiée le contenant

(43) Date of publication of application: 02.01.2008
(73) Proprietor: Sumitomo Chemical Company, Limited, Tokyo 104-8260 (JP)
(72) Inventor: Yoshida, Isao, Ikeda-shi Osaka (JP); Harada, Yukako, Settu-shi Osaka (JP); Yamaguchi, Satoshi, Toyonaka-shi Osaka (JP); Ando, Nobuo, Toyonaka-shi Osaka (JP)
(74) Representative: Vossius & Partner

(56) References cited:
- EP-A1- 1 077 391
- JP-A- 2004 004 561

## Description

The present invention relates to a salt suitable for an acid generator useful for a chemically amplified resist composition which is useful in fine processing of semiconductors, and a chemically amplified resist composition containing the salt.

A chemically amplified resist composition used for semiconductor microfabrication employing a lithography process contains an acid generator comprising a compound generating an acid by irradiation.

In semiconductor microfabrication, it is desirable to form patterns having improved pattern profiles and having high resolution and, and it is expected for a chemically amplified resist composition to give such patterns.

Recently, a chemically amplified resist composition containing triphenylsulfonium 1-adamantanemethoxycarbonyldifluoromethanesulfonate, p-tolyldiphenylsulfonium perfluorooctanesulfonate, and the like, and a salt providing a chemically amplified resist composition giving patterns having better profiles and higher resolution was proposed (e.g. US6 893 792).

Objects of the present invention are to provide a salt suitable for an acid generator capable of providing chemically amplified resist compositions giving patterns having better resolution and having improved pattern profiles and a process for producing the salt.

Another objects of the present invention are to provide synthetic intermediates for the salts and to provide a process for producing the synthetic intermediates or the salts.

Still another object of the present invention is to provide a chemically amplified resist composition containing the salts.

These and other objects of the present invention will be apparent from the following description.

The present invention relates to the followings :
<1> A salt of the formula (L): 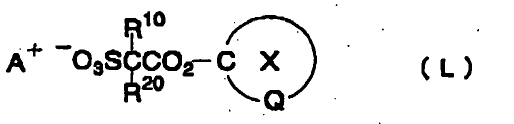 wherein Q represents -CO-group or-C(OH)- group; ring X represents monocyclic or polycyclic hydrocarbon group having 3 to 30 carbon atoms in which a hydrogen atom is substituted by a hydroxyl group at Q position when Q is -C(OH)- group or in which two hydrogen atoms are substituted by =O group at Q position when Q is -CO group, and at least one hydrogen atom in the monocyclic or polycyclic hydrocarbon group may optionally be substituted by an alkyl group having 1 to 6 carbon atoms, alkoxy group having 1 to 6 carbon atoms, perfluoroalkyl group having 1 to 4 carbon atoms, hydroxyalkyl group having 1 to 6 carbon atoms, hydroxyl group or cyano group; R¹⁰ and R²⁰ each independently represent fluorine atom or perfluoroalkyl group having 1 to 6 carbon atoms; and A⁺ represents organic counter ion. Hereinafter, the salt of the formula (L) may be referred to as Salt (L).
<2> The salt according to <1>, wherein each of R¹⁰ and R²⁰ is fluorine atom or trifluoromethyl group.
<3> The salt according to <1> or <2>, wherein the salt has the following formula (I) 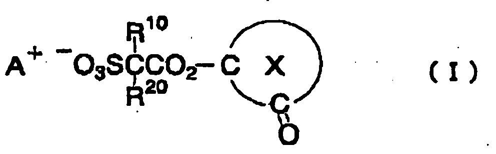 wherein X,A,R¹⁰ and R²⁰ have the same meanings as defined above.
<4> The salt according to <1> or <2>, wherein the salt bas the following formula (II) 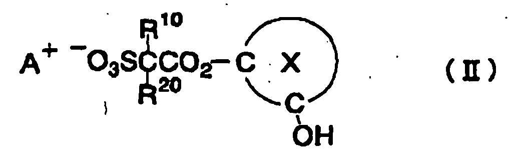 wherein X, A, R¹⁰ and R²⁰ have the same meanings as defined above.
<5> The salt according to any of <1> to <4>, wherein A⁺ is at least one cation selected from the group consisting of the formula (IIIa), the formula (IIIb), the formula (IIIc) and the formula (IIId).
   A cation of the formula (IIIa) 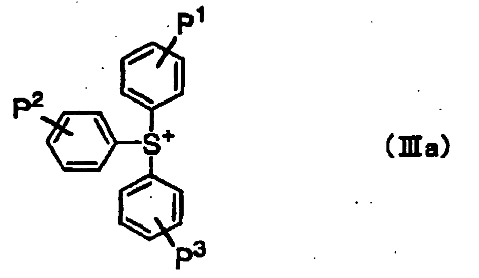 wherein P¹, P² and P³ each independently represent hydrogen atom, hydroxyl group, alkyl group having 1 to 12 carbon atoms or alkoxy group having 1 to 12 carbon atoms.
   A cation of the formula (IIIb) 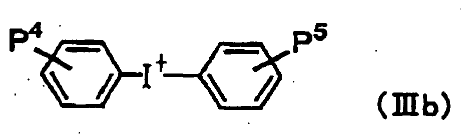 wherein P⁴ and P⁵ each independently represent hydrogen atom, hydroxyl group, alkyl group having 1 to 12 carbon atoms or alkoxy group having 1 to 12 carbon atoms.
   A cation of the Formula (IIIc) 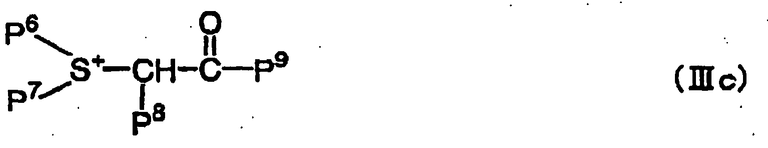 wherein P⁶ and P⁷ each independently represent alkyl having 1 to 12 carbon atoms or cycloalkyl having 3 to 12 carbon atoms, or P⁶ and P⁷ bond to form divalent acyclic hydrocarbon group having 3 to 12 carbon atoms which forms a ring together with the adjacent S⁺, and at least one-CH₂-in the divalent acyclic hydrocarbon group is optionally substituted by -CO-, -O- or -S-, P⁸ represents hydrogen, P⁹ represents alkyl having 1 to 12 carbon atoms, cycloalkyl having 3 to 12 carbon atoms or aromatic ring group optionally substituted, or P⁸ and P⁹ bond to form divalent acyclic hydrocarbon group which forms 2-oxocycloalkyl together with the adjacent -CHCO-, and at least one -CH₂- in the divalent acyclic hydrocarbon group is optionally substituted by -CO-, -O- or -S-.
   A cation of the formula (IIId) 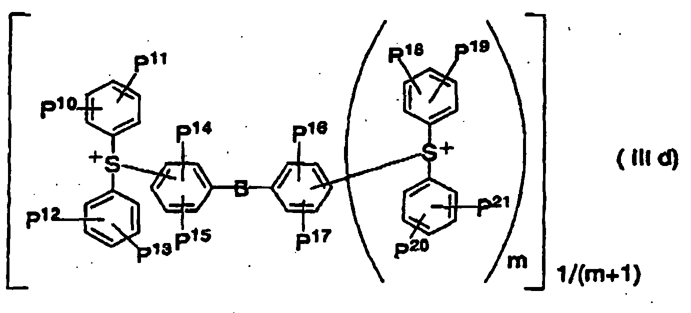 Wherein P¹⁰,P¹¹,P¹²,P¹³,P¹⁴,P¹⁵,P¹⁶,P¹⁷,P¹⁸,P¹⁹,P²⁰ and P²¹ each independently represent hydrogen atom, hydroxyl group, alkyl group having 1 to 12 carbon atoms or alkoxy group having 1 to 12 carbon atoms, B represents sulfur atom, and m represents 0 or 1.
<6> The salt according to any of any of <1> to <5>, wherein A⁺ is a counter ion of the formula (IIIe): 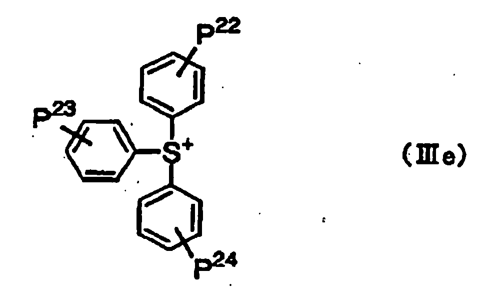 wherein P²², P²³ and P²⁴ each independently represent hydrogen atom or alkyl group having 1 to 4 atoms,
**<7>** The salt according to any of <1> to <6>, wherein the ring X is cycloalkyl group having 4 to 8 carbon atoms, adamantyl group or norbornyl group, provided that, in each of the groups, a hydrogen atom is substituted by -OH group at Q position when Q is -C(OH)- group and two hydrogen atoms are substituted by =O at Q position when Q is -CO- group, and provided that at least one hydrogen atom in each of the group may optionally be substituted by an alkyl group having 1 to 6 carbon atoms, alkoxy group having 1 to 6 carbon atoms, perfluoroalkyl group having 1 to 4 carbon atoms, hydroxyalkyl group having 1 to 6 carbon atoms, hydroxyl group or cyano group.
<8> The salt according to <6>, wherein the salt is the one of the formula (IVa) 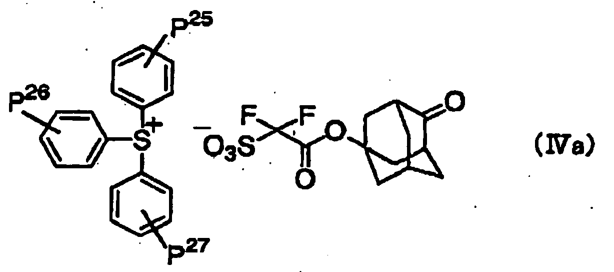 wherein P²⁵, P²⁶ and P²⁷ each independently represent hydrogen atom or alkyl group having 1 to 4 carbon atoms.
<9> The salt according to <6>, wherein the salt is the one of the formula (IVb) 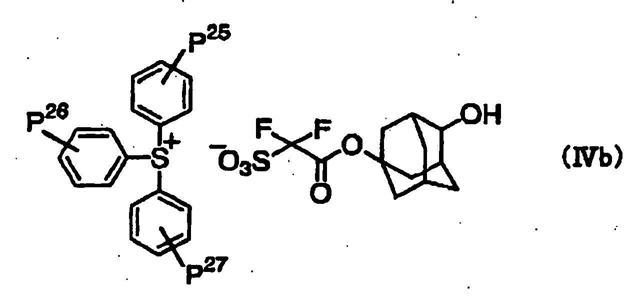 wherein P²⁵,P²⁶ and P²⁷ have the same meanings as defined above.
<10> A salt of the formula (LI) 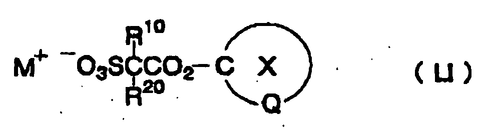 wherein Q, X, R¹⁰ and R²⁰ have the same meanings as defined above, and M represents Li, Na, K or Ag.
<11> The salt according to <10>, wherein the salt has the formula (V) 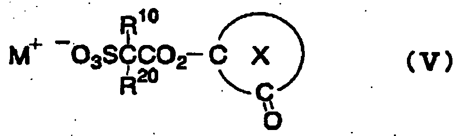 wherein X, R¹⁰, R²⁰ and M have the same as defined above.
<12> The salt according to <10>, wherein the salt has the formula (VI) 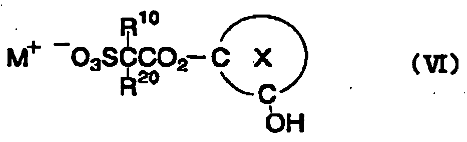 where X, R¹⁰, R²⁰, and M have the same meanings as defined above.
<12-1> The salt according to any of <10> to <12>, wherein each of R¹⁰ and R²⁰ is fluorine atom or trifluoromethyl group.
<13> A process for producing a salt of the formula (LI), which comprises esterifying an alcohol of the formula (LII) 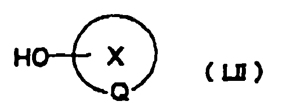 wherein X and Q have the same meanings as defined above,
   with a carboxylic acid of the formula (IX) 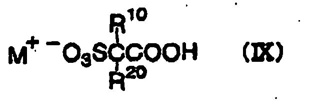 wherein R¹⁰, and M have the same meanings as defined above.
<14> A process for producing a salt of the formula (II), which comprises reducing a salt of the formula (I).
<15> A process for producing a salt of the formula (VI), which comprises reducing a salt of the formula (V).
<16> A process for producing a salt of the formula (LI), which comprises esterifying an alcohol of the formula (LII) with a carboxylic acid of the formula (X) 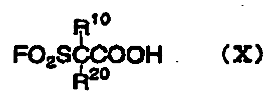 wherein R¹⁰ and R²⁰ have the same meanings as defined above,
   and hydrolyzing with MOH,
   wherein M has the same meaning as defined above.
<17> A process for producing Salt (L), which comprises reacting a salt of the formula (LI) with a compound of the formula (XI)

   A⁺Z*⁻* (XI)

   wherein Z represents F, Cl, Br, I, BF₄, AsF₆, SbF₆, PF₆ or ClO₄, and A⁺ has the same meaning as defined above.
<17-1> The process according to any of <13> to <17>, wherein each of R¹⁰ and R²⁰ is fluorine atom or trifluoromethyl group.
<18> A chemically amplified resist composition comprising Salt (L) and
   a resin which contains a structural unit having an acid-labile group and which itself is insoluble or poorly soluble in an alkali aqueous solution but becomes soluble in an alkali aqueous solution by the action of an acid.
<19> The composition according to <18>, wherein each of R¹⁰ and R²⁰ is fluorine atom or trifluoromethyl group.
<20> The composition according to <18> or <19>, wherein the resin contains a structural unit derived from a monomer having a bulky and acid-labile group.
<21> The composition according to <20>, wherein the bulky and acid-labile group is 2-alkyl-2-adamantyl group or 1-(1-adamantyl)-1-alkyalkyl group.
<22> The composition according to <20>, wherein the monomer having bulky and acid-labile group is 2-alkyl-2-adamantyl (meth)acrylate,1-(1-adamantyl)-1-alkylalkyl (meth)acrylate, 2-alkyl-2-adamantyl 5-norbomene-2-carboxylate, 1-(1-adamantyl)-1-alkylalkyl 5-norbomene-2-carboxylate, 2-alkyl-2-adamantyl α-chloroacrylate or 1-(1-adamantyl)-1-alkylalkyl α-chloroacrylate.
<23> The composition according to any of <18> to <22>, wherein the composition further comprises a basic compound.
<24> The composition according to any of <18> to <23>, wherein the salt is the one of the formula (I).
<25> The composition according to any of <18> to <23>, wherein the salt is the one of the formula (II).
<26> The composition according to any of <18> to <25>, wherein A⁺ is at least one cation selected from the group consisting of the formula (IIIa), the formula (IIIb), the formula (IIIc) and the formula (IIId).
<27> The composition according to <26>, wherein A⁺ is a counter ion of the formula (IIIe).
<28> The composition according to any of <18> to <27>, wherein the ring X is cycloalkyl group having 4 to 8 carbon atoms, adamantyl group or norbornyl group, provided that, in each of the groups, a hydrogen atom is substituted by -OH group at Q position when Q is -C(OH)-group and two hydrogen atoms are substituted by =O at Q position when Q is-CO- group, and provided that at least one hydrogen atom in each of the group may optionally be substituted by an alkyl group having 1 to 6 carbon atoms, alkoxy group having 1 to 6 carbon atoms, perfluoroalkyl group having 1 to 4 carbon atoms, hydroxyalkyl group having 1 to 6 carbon atoms, hydroxyl group or cyano group.
<29> The composition according to <27>, wherein the salt is the one of the formula (IVa).
<30> The composition according to <27> wherein the salt is the one of the formula (IVb).

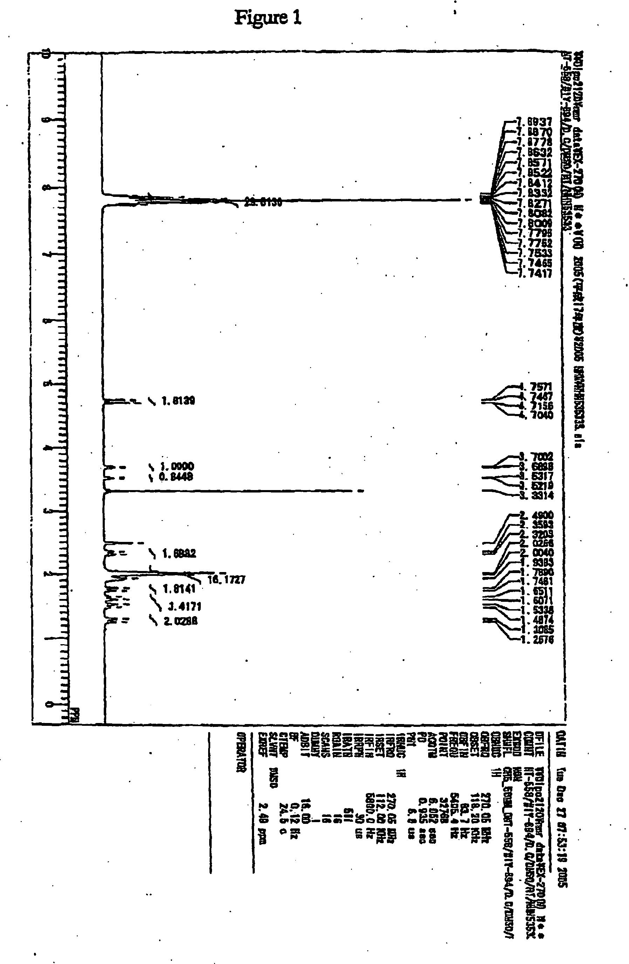

Figure 1 is the ¹H-NMR spectrum chart of triphenylsulfonium 4-hydroxy-1-adamantyloxycarbonyldifluoromethanesulfonate produced in Synthetic Example 2.

In Salt (L), Q represents -CO- group or -C(OH)- group. when Q is -CO- group, the salt is Salt (I), and when Q is -C(OH)- group, the salt is Salt (II).

When Q is -CO- group, the ring X represents monocyclic or polycyclic hydrocarbon group having 3 to 30 carbon atoms in which two hydrogen atoms at Q position are substituted by=O. When Q is -C(OH)-group, the ring X represents a monocyclic or polycyclic hydrocarbon group having 3 to 30 carbon atoms in which a hydrogen atom at Q position is substituted by a hydroxyl group. At least one hydrogen atom in the monocyclic or polycyclic hydrocarbon group may optionally be substituted by an alkyl group having 1 to 6 carbon atoms, alkoxy group having 1 to 6 carbon atoms, perfluoroalkyl group having 1 to 4 carbon atoms, hydroxyalkyl group having 1 to 6 carbon atoms, hydroxyl group or cyano group.

Examples of the ring X include cycloalkyl group having 4 to 8 carbon atoms, adamantyl group or norbornyl group, wherein, in each of the cycloalkyl group, adamantyl group and norbornyl group, a hydrogen atom is substituted by -OH group at Q position when Q is -C(OH)- group and two hydrogen atoms are substituted by =O at Q position when Q is -CO-group, and at least one hydrogen atom in each of the cycloalkyl group, adamantyl group and norbornyl group may optionally be substituted by an alkyl group having 1 to 6 carbon atoms, alkoxy group having 1 to 6 carbon atoms, perfluoroalkyl group having 1 to 4 carbon atoms, hydroxyalkyl group having 1 to 6 carbon atoms, hydroxyl group or cyano group.

Specific examples of the ring X include 2-oxocyclopentyl group, 2-oxocyclohexyl group, 3-oxocyclopentyl group, 3-oxocyclohexyl group, 4-oxocyclohexyl group, 2-hydroxycyclopentyl group, 2-hydroxycyclohexyl group, 3-hydroxycyclopentyl group, 3-hydroxycydohexyl group, 4-hydroxycyclohexyl group, 4-oxo-2-adamantyl group, 3-hydroxy-1-adamantyl group, 4-hydroxy-1-adamantyl group, 5-axonorboman-2-yl group, 1,7,7-trimethyl-2-oxonorbornan-2-yl group, 3,6,6-trimethyl-2-oxo-bicyclo[3.1.1]heptan-3-yl group, 2-hydroxynorbornan-3-yl group, 1,7,7-trimethyl-2-hydroxynorbornan-3-yl group, 3,6,6-trimethyl-2-hydroxybicyclo[3.1.1]heptan-3-yl group, 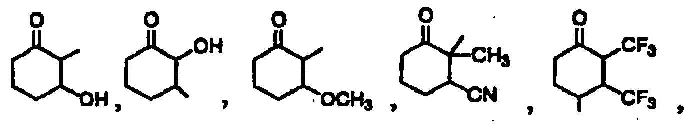 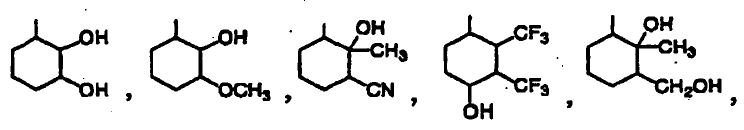 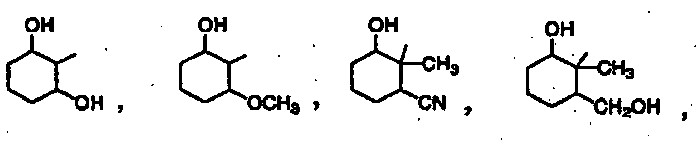 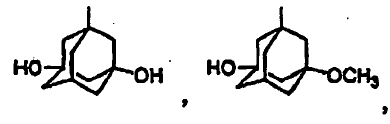 and the like.
In the formulae above, straight line with an open end shows a bond which is extended from an adjacent group.

R¹⁰ and R²⁰ each independently represent fluorine atom or perfluoroalkyl group having 1 to 6 carbon atoms such as trifluoromethyl group, pentafluoroethyl group, heptafluoropropyl group, nonafluorobutyl group, undecafluoropentyl group, tridecafluorohexyl group, and the like.

Specific examples of anion part of the salt (L) include the following :

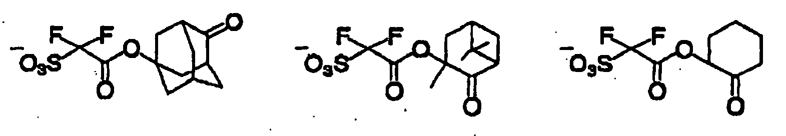

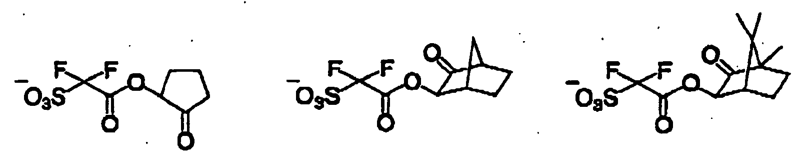

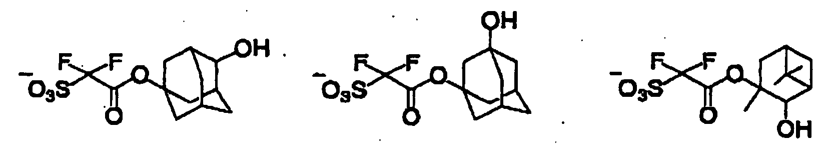

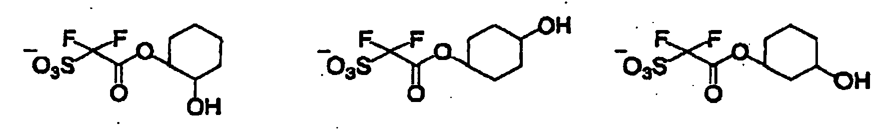

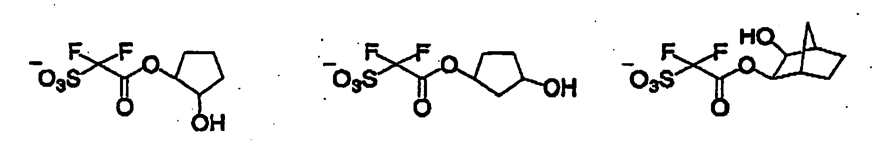

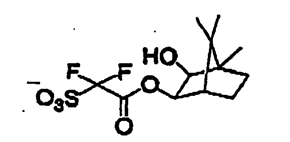

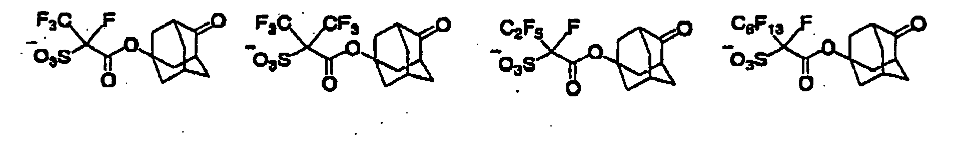

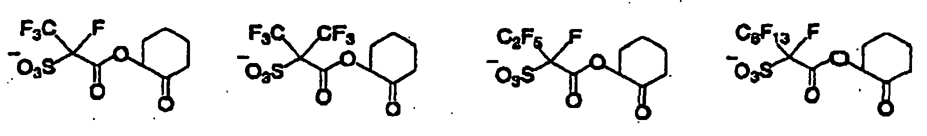

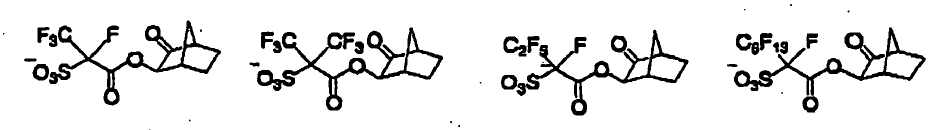

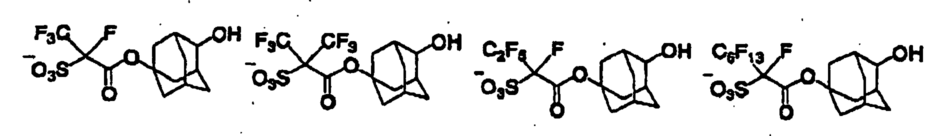

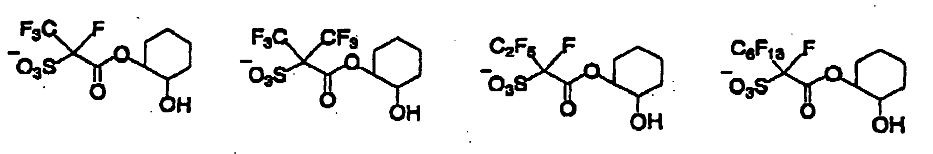

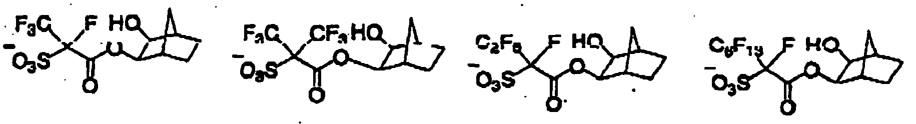

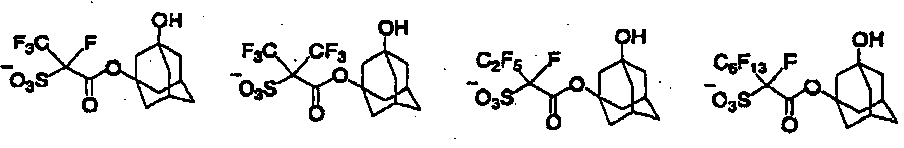

A⁺ in Salt (L) represents an organic counter ion. Examples thereof include the following cations of the formulae (IIIa), (IIIb), (IIIc) and (IIId).

In the cation of the formula (IIIa), P¹, P² and P³ each independently represent hydrogen atom, hydroxyl group, alkyl group having 1 to 12 carbon atoms or alkoxy group having 1 to 12 carbon atoms.

Examples of the alkyl group in the formula (IIIa) include methyl group, ethyl group, propyl group, isopropyl group, butyl group, tert-butyl group, pentyl group, hexyl group, octyl group, 2-ethylhexyl group, and the like, and examples of the alkoxy group include methoxy group, ethoxy group, propoxy group, butoxy group, hexyloxy group, octyloxy group, 2-ethylhexyloxy group, and the like.

In the cation of the formula (IIIa), the one of the formula (IIIe) above is preferred for the easiness of production.

In the cation of the formula (IIIb), P⁴ and P⁵ each independently represent hydrogen atom, hydroxyl group, alkyl group having 1 to 12 carbon atoms or alkoxy group having 1 to 12 carbon atoms. Examples of the alkyl group and alkoxy group include the same groups as mentioned in the formula (IIIa) above.

In the cation of the formula (IIIc), P⁶ and P⁷ each independently represent alkyl having 1 to 12 carbon atoms or cycloalkyl having 3 to 12 carbon atoms, or P⁶ and P⁷ bond to form divalent acyclic hydrocarbon group having 3 to 12 carbon atoms which forms a ring together with the adjacent S⁺, and at least one -CH₂- in the divalent acyclic hydrocarbon may be substituted by -CO-, -0- or -S-.

P⁸ represents hydrogen, P⁹ represents alkyl having 1 to 12 carbon atoms, cycloalkyl having 3 to 12 carbon atoms or aromatic ring group optionally substituted, or P⁸ and P⁹ bond to form divalent acyclic hydrocarbon group which forms 2-oxocycloalkyl together with the adjacent -CHCO-, and at least one -CH₂- in the divalent acyclic hydrocarbon group may be substituted by -CO-, -O- or -S-.

In P⁶, P⁷ and P⁹, specific examples of the alkyl group include methyl group, ethyl group, propyl group, isopropyl group, butyl group, tert-butyl group, pentyl group, hexyl group, and the like, and specific examples of the cycloalkyl group include cyclopropyl group, cyclobutyl group, cyclopentyl group, cyclohexyl group, cycloheptyl group, cyclodecyl group, and the like. Specific examples of the divalent acyclic hydrocarbon group having 3 to 12 carbon atoms formed by bonding P⁶ and P⁷ include methylene group, ethylene group, trimethylene group, tetramethylene group, pentamethylene group, and the like, and specific examples of the ring group formed by adjacent S⁺ and divalent acyclic hydrocarbon group by P⁶ and P⁷ include pentamethylenesulfonio group, tetramethylenesulfonio group, oxybisethylenesulfonio group, and the like. In P⁹, specific examples of the aromatic ring group include phenyl, tolyl, xylyl, naphtyl and the like. Specific examples of the divalent acyclic hydrocarbon group formed by bonding P⁸ and P⁹ include methylene group, ethylene group, trimethylene group, tetramethylene group, pentamethylene group, and the like, and specific examples of the 2-oxocycloalkyl formed by bonding P⁸ and P⁹ together with the adjacent CHCO-include 2-oxocyclohexyl, 2-oxocyclopentyl and the like.

In the cation of the formula (IIId), P¹⁰, P¹¹, P¹², P¹³, P¹⁴, P¹⁵, P¹⁶, P¹⁷, P¹⁸, P¹⁹, P²⁰ and P²¹ each independently represent hydrogen atom, hydroxyl group, alkyl group having 1 to 12 carbon atoms or alkoxy group having 1 to 12 carbon atoms, B represents sulfur atom or oxygen atom, and m represents 0 or 1. Examples of the alkyl group and alkoxy group include the same groups as mentioned in the formula (IIIa) above.

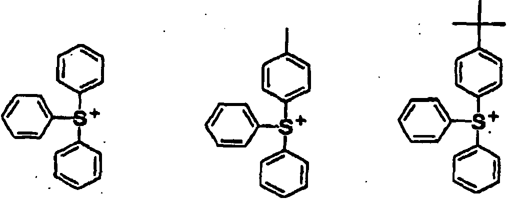

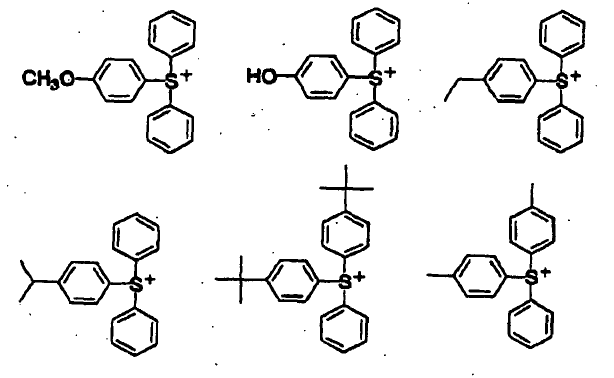

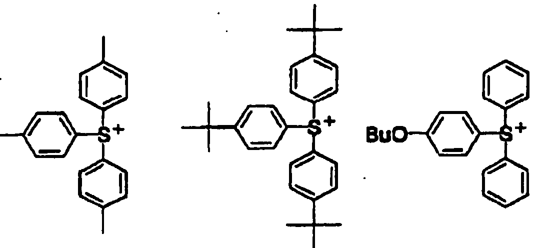

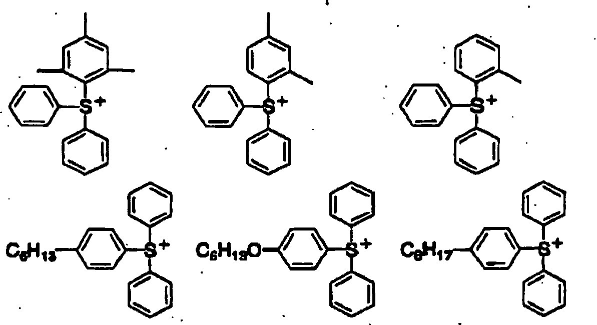

Specific examples of the formula (IIIb) include the following ;

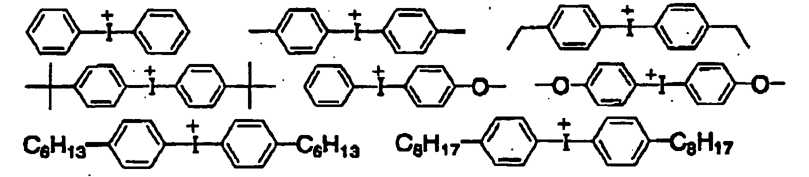

Specific examples of the formula (IIIc) include the following:

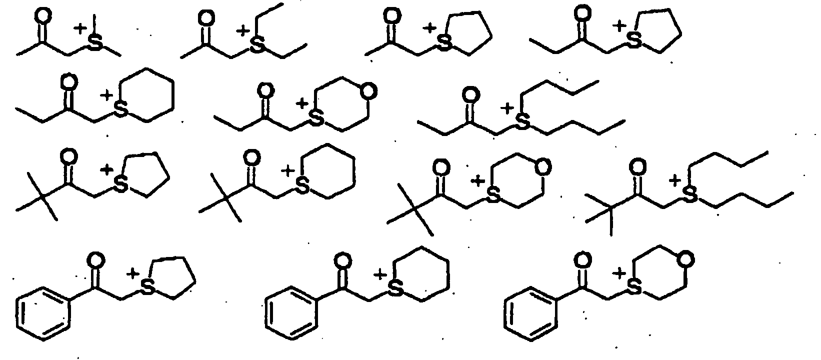

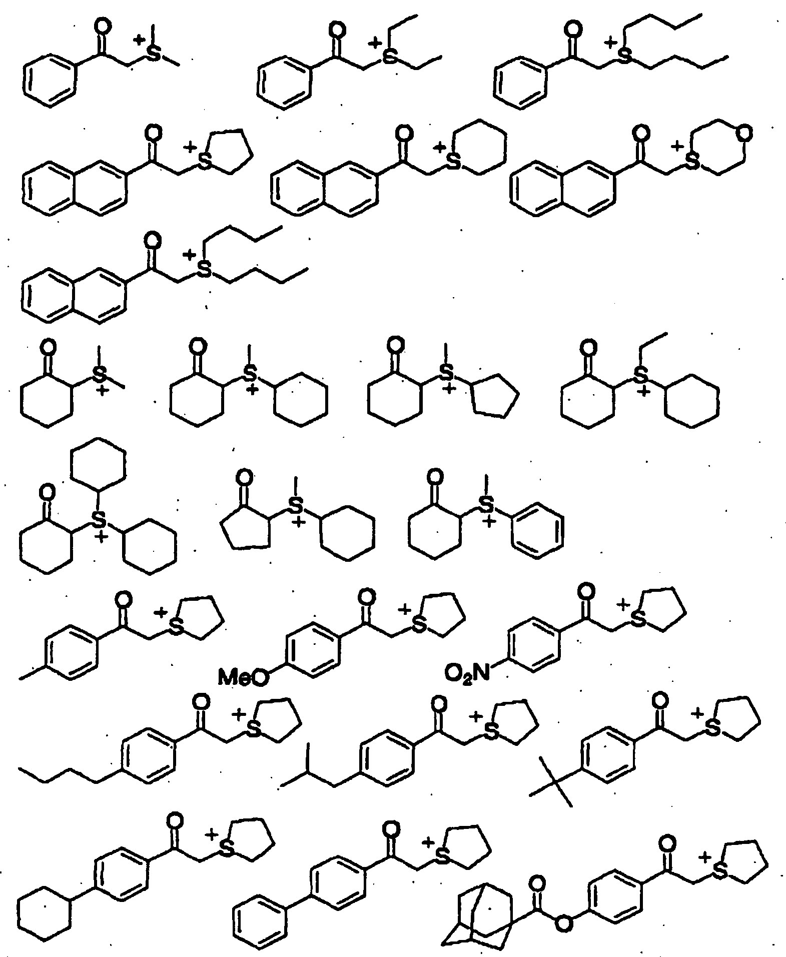

Specific examples of the cation of the formule (IIId) include the following :

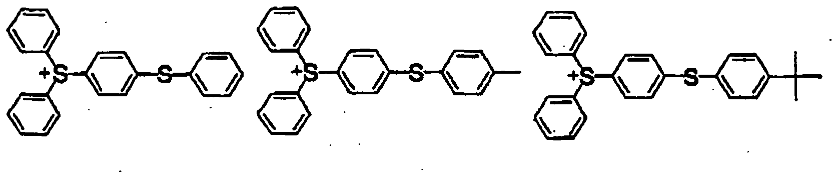

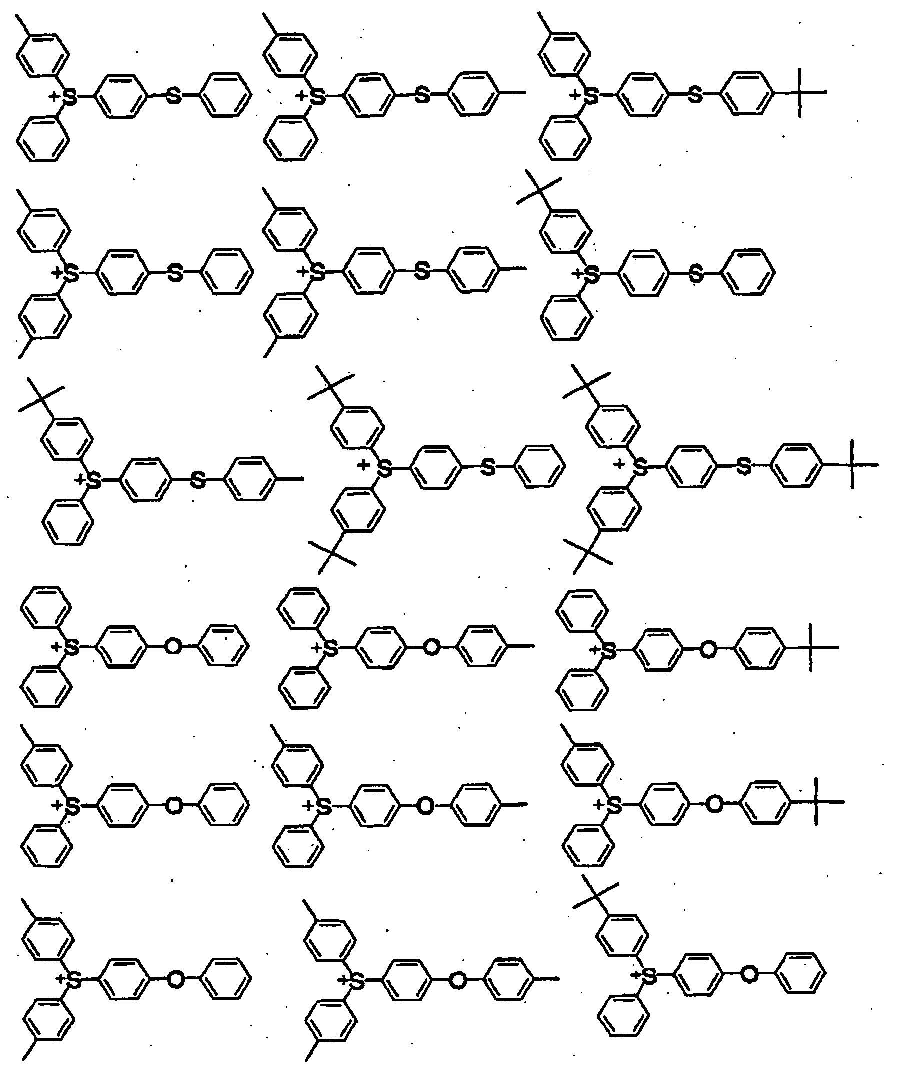

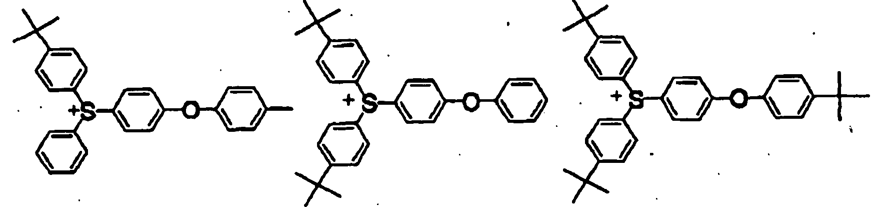

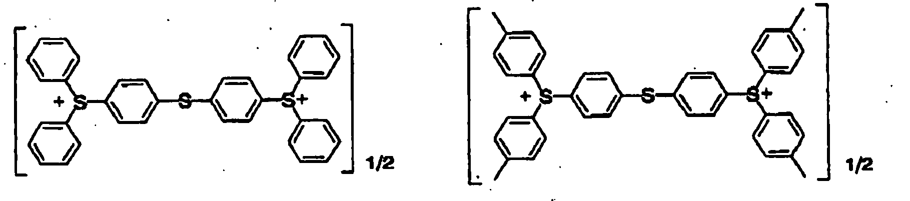

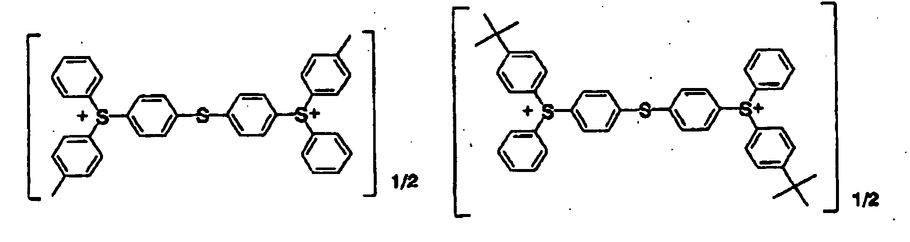

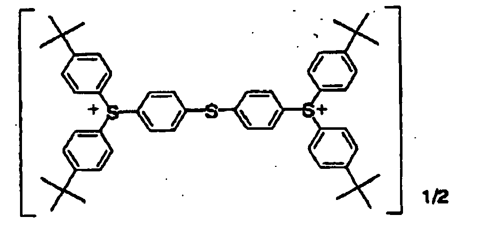

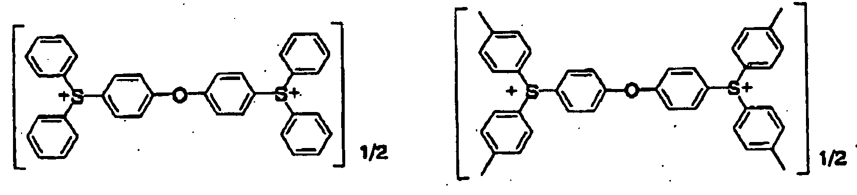

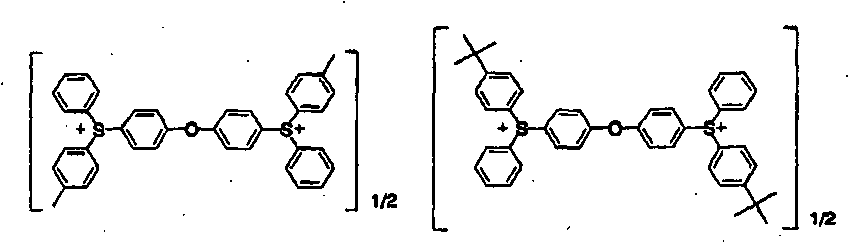

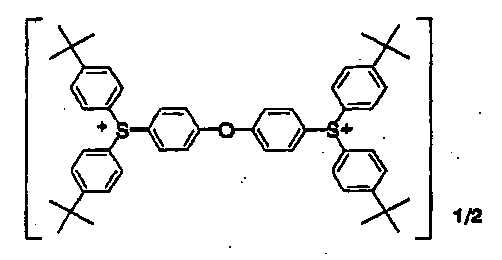

As Salt (L), the salts of the formulae (IVa) and (IVb) are preferred for the excellent resolution and pattern profile. 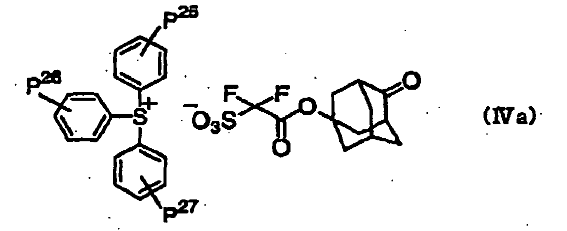 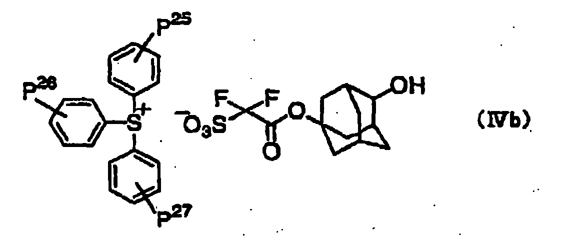 In the formule (IVa) and (IVb), P²⁵, P²⁶ and P²⁷ each independently represent hydrogen atom or alkyl group having 1 to 4 carbon atom.

Examples of the process for production of Salt (L) include a process comprising reacting a salt of the formula (LI) with a compound of the formula (XI), for example, in an inert solvent such as acetonitrile, water, methanol, dichloromethane, and the like, at a temperature of about 0 to 150°C, preferably of 0 to 100°C, with stirring.

The amount of the compound of the formula (XI) is usually 0.5 to 2 mols per 1 mol of the salt of the formula (LI). Salt (L) obtained by the process above can be isolated by recrystallization, and can be purified by washing with water.

The salt of the formula (LI) used for the production of Salt (L) can be produced for example, by a process comprising esterifying an alcohol of the formula (LII) with a carboxylic acid of the formula (IX).

When Q is -CO-, the alcohol of the formula (LII) is an alcohol of the formula (VII) 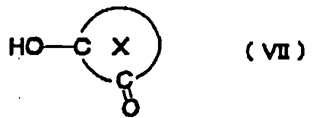 wherein X has the same meaning as defined above, and
when Q is -C(OH)-, the alcohol of the formula (LII) is an alcohol of the formula (VIII) 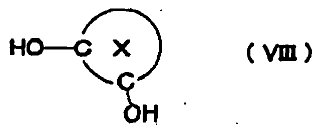 wherein X has the same meaning as defined above.

The esterification reaction can generally be carried out by mixing materials in an aprotic solvent such as dichloroethane, toluene, ethylbenzene, monochlorobenzene, acetonitrile, N,N-dimethylformamide, and the like, at 20 to 200°C, preferably 50 to 150°C. In the esterification reaction, an acid catalyst or a dehydrating agent is usually added, and examples of the acid catalyst include organic acids such as p-toluenesulfonic acid, inorganic acids such as sulfuric acid, and the like. Examples of the dehydrating agent include 1,1'-carbonyldiimidazole, N,N'-dicyclohexylcarbodiimide, and the like.

The esterification may preferably be carried out with dehydration, for example, by Dean and Stark method as the reaction time tends to be shortened.

The amount of the carboxylic acid of the formula (IX) is usually 0.2 to 3 mol, preferably 0.5 to 2 mol per 1 mol of the alcohol of the formula (LII). The amount of the acid catalyst may be catalytic amount or the amount equivalent to solvent, and is usually 0.001 to 5 mol per 1 mol of the alcohol of the formula (LII). The amount of the dehydrating agent is usually 0.2 to 5 mol, preferably 0.5 to 3 mol per 1 mol of the alcohol of the formula (LII).

The salt of the formula (LI) can also be produced by a process comprising esterifying the alcohol of the formula (LII) with a carboxylic acid of the formula (X), then hydrolyzing the esterified compound with MOH, wherein M represents Li, Na, K or Ag. The esterification reaction can be carried out in the same manner as in the esterification of the alcohol of the formula (LII) with carboxylic acid of the formula (IX). The hydrolyzation reaction can usually be carried out by stirring a mixture of the esterified compound obtained by the esterification reaction and MOH in a solvent. The solvent may be water or a mixed solvent of water and water-soluble solvent such as acetonitrile, tetrahydrofuran, and the like. The end point of the reaction can be determined, for example by the analysis of ¹⁹FNMR. The amount of the carboxylic acid of the formula (X) is usually 0.2 to 3 mol, preferably 0.5 to 2 mol per 1 mol of the alcohol of the formula (LII). The amount of the acid catalyst may be catalytic amount or the amount equivalent to solvent, and is usually 0.001 to 5 mol per 1 mol of the alcohol of the formula (LII). The amount of the dehydrating agent is usually 0.2 to 5 mol, preferably 0.5 to 3 mol per 1 mol of the alcohol of the formula (LII).

The amount of the MOH is usually 02 to 3 mol, preferably 1 to 2 mol per 1 mol of the ester.
The salt of the formula (II) can also be produced by reducing the salt of the formula (I), and similarly the salt of the formula (VI) can also be produced by reducing the salt of the formula (V).

The reduction reaction can be carried out by using reducing agents such as boron hydride compounds (e.g. sodium borohydride, zinc borohydride, lithium trisec.-butylborohydride, borane, and the like), aluminum hydride compounds (e.g. lithium tri-tert-butoxyalminum hydride, diisobutylaluminum hydride, and the like), organosilicon hydride compounds (e.g. Et₃SiH, Ph₂SiH₂, and the like), and the like, in a solvent such as water, alcohol, acetonitrile, N,N-dimethylformamide, diglyme, tetrahydrofuran, diethyl ether, dichloromethane, 1,2-dimethoxyethane, benzene, and the like. Reaction is usually carried out at a temperature of -80 to +100°C, preferably at -10 to +60°C with stirring.

The present chemically amplified resist composition comprises Salt (L) and a resin which contains a structural unit having an acid-labile group and which itself is insoluble or poorly soluble in an alkali aqueous solution but becomes soluble in an alkali aqueous solution by the action of an acid.

Salt (L) is usually used as an acid generator, and the acid generated by irradiation to Salt (L) catalytically acts against acid-labile groups in a resin, cleaves the acid-labile-group, and the resin becomes soluble in an alkali aqueous solution. Such a composition is suitable for chemically amplified positive resist composition.

The resin used for the present composition contains a structural unit having an acid-labile group and which itself is insoluble or poorly soluble in an alkali aqueous solution, but acid-labile group cleave by an acid. The resin after the cleavage contains carboxylic acid residue and as a result, the resin becomes soluble in an alkali aqueous solution.

In the present specification, "-COOR" may be described as "a structure having ester of carboxylic acid", and may also be abbreviated as "ester group". Specifically, "-COOC(CH₃)₃" may be described as "a structure having tert-butyl ester of carboxylic acid", or be abbreviated as "tert-butyl ester group".

Examples of the acid-labile group include a structure having ester of carboxylic acid such as alkyl ester group in which a carbon atom adjacent to the oxygen atom is quaternary carbon atom, and alicyclic ester group in which a carbon atom adjacent to the oxygen atom is quaternary carbon atom, and the like, a lactone ring group in which a carbon atom adjacent to the oxygen atom is quaternary carbon atom, and the like.

The "quaternary carbon atom "means a" carbon atom joined to four substituents other than hydrogen atom". As the acid-labile group, a group having a quaternary carbon atom joined to three carbon atoms and a OR', wherein R' represents alkyl group.

Examples of the acid-labile group include alkyl ester group in which a carbon atom adjacent to the oxygen atom is quaternary carbon atom such as tert-butyl ester group; acetal type ester group such as methoxymethyl ester group, ethoxymethyl ester group, 1-ethoxyethyl ester group, 1-isobutoxyethyl ester group, 1-isopropoxyethyl ester group, 1-ethoxypropoxy ester group, 1-(2-methoxyethoxy)ethyl ester, 1-(2-acetoxyethoxy)ethyl ester group, 1-[2-(1-adamantyloxy)ethoxy]ethyl ester group, 1-[2,(1-adamantanecarbonyloxy)ethoxy]ethyl ester group, tetrahydro-2-furyl ester and tetrahydro-2-pyranyl ester group; alicyclic ester group in which a carbon atom adjacent to the oxygen atom is quaternary carbon atom, such as isobomyl ester group, 1-alkylcycloalkyl ester group, 2-alkyl-2-adamantyl ester group, 1-(1-adamantyl)1-alkylalkyl ester group, and the like.

Examples of structures including the ester group include ester of (meth)acrylic acid structure, ester of norbornenecarboxylic acid structure, ester of tricyclodecenecarboxylic acid structure, tetracyclodecenecarboxylic acid structure, and the like. At least one hydrogen atom in the adamantyl group above may be substituted by hydroxyl group.

The resin used for the present composition can be obtained by addition polymerization of monomer(s) having an acid-labile group and olefinic double bond.

Among the monomers, it is preferable to use those having a bulky group such as alicyclic group (e.g. 2-alkyl-2-adamantyl and 1-(1-adamantyl)-1-alkylalkyl), as the group dissociated by the action of an acid, since excellent resolution is obtained when used in the present composition.

Examples of such monomer containing a bulky group include 2-alkyl-2-adamantyl (meth)acrylate,1-(1-adamantyl)-1-alkylalkyl (meth)acrylate, 2-alkyl-2-adamantyl5-norbomene-2-carboxylate,1-(1-adamantyl)-1-alkylalkyl 5-norbornene-2-carboxylate, 2-alkyl-2-adamantyl α-chloroacrylate, 1-(1-adamantyl)-1-alkylalkyl α-chloroacrylate and the like.

In this context, alkyl is typically a C₁₋₆ alkyl, preferably a C₁₋₄ alkyl.

Particularly when 2-alkyl-2-admantyl (meth)acrylate or 2-alkyl-2-ademantyl α-chloroacrylate is used as the monomer for the resin component in the present composition, resist composition having excellent resolution tend to be obtained Typical examples of such 2-alkyl-2-adamantyl (meth)acrylate and 2-alkyl-2-adamantyl α-chloroacrylate include 2-methyl-2-adamantyl acrylate, 2-methyl-2-adamantyl methacrylate, 2-ethyl-2-adamantyl acrylate, 2-ethyl-2-adamantyl methacrylate, 2-n-butyl-2-adamantyl acrylate, 2-methyl-2-adamantyl α-chloroacrylate, 2-ethyl-2-adamantyl α-chloroacrylate and the like. When particularly 2-ethyl-2-adamantyl (meth)acrylate or 2-isopropyl-2-adamantyl (meth)acrylate is used for the present composition, composition having excellent sensitivity and heat resistance tends to be obtained. In the present invention, two or more kind of monomers having group dissociated by the action of an acid may be used together, if necessary.

2-Alkyl-2-adamantyl (meth)acylate can usually be produced by reacting 2-alkyl-2-adamantanol or metal salt thereof with an acrylic halide or methacrylic halide,

The resin used for the present composition can also contain, in addition to the above-mentioned structural units having an acid-labile group, other structural units derived from acid-stable monomer. Herein, the "structural unit derived from acid-stable monomer" means "a structural unit not dissociated by an acid generated form Salt (L)".

Examples of such other structural units which can be contained include structural units derived from monomers having a free carboxyl group such as acrylic acid and methacrylic acid, structural units derived from aliphatic unsaturated dicarboxylic anhydrides such as maleic anhydride and itaconic anhydride, structural units derived from 2-norbornene, structural units derived from (meth)acrylonitrile, structural units derived from alkyl (meth)acrylate in which a carbon atom adjacent to oxygen atom is secondary or tertiary carbon atom, structural units derived from 1-adamantyl(meth)acrylate, structural units derived from styrenes such as p- or m-hydroxystyrene, structural units derived from (meth)acryloyloxy-γ-butyrolactone having a lactone ring optionally substituted by alkyl, and the like. Herein, 1-adamatyl ester group is a acid-stable group though the carbon atom adjacent to oxygen atom is a quaternary carbon atom, and at least one hydrogen atom on 1-adamantyl ester group may be substituted by hydroxy group.

Specific examples of structural unit derived from acid-stable monomer include a structural unit derived from 3-hydroxyl-1-adamantyl (meth)acrylate, a structural unit derived from 3,5-dihydroxy-1-adamatyl (meth)acrylate, a structural unit derived from α-(meth)acryloyloxy-γ-butyrolactone, a structural unit derived from β-(meth)acryloyloxy-γ-butyrolactone, a structural unit of the following formula (a), a structural unit derived from the following formula (b), a structural unit derived from alicyclic compound having olefinic double bond such as a structural unit of the following formula (c), a structural unit derived from aliphatic unsaturated dicarboxylic anhydride such as a structural unit of the formula (d), a structural unit of the formula (e), and the like.

Particularly, to contain, in addition to the structural unit having an acid-labile group, further at least one structural unit selected from the group consisting of a structural unit derived from p-hydroxystyrene, a structural unit derived from m-hydroxystyrene, a structural unit derived from 3-hydroxy-1-adamantyl (meth) acrylate, a structural unit derived from 3,5-dihydroxy-1-adamantyl (meth)acrylate, a structural unit of the following formula (a) and a structural unit of the following formula (b), in the resin in the present composition, is preferable from the standpoint of the adhesiveness of resist to a substrate and resolution of resist. 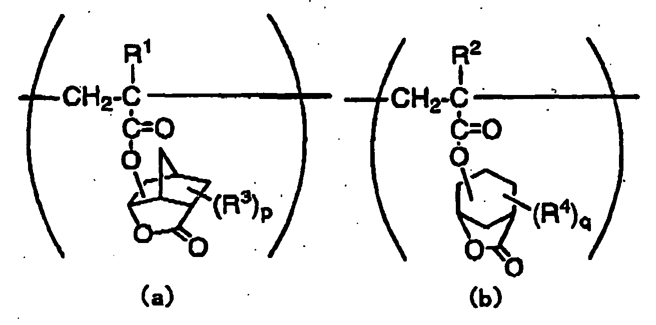 In the formulae (a) and (b), R¹ and R² each independently represent hydrogen atom, methyl group or trifluoromethyl group and R³ and R⁴ each independently represent methyl group, trifluoromethyl group or halogen atom, and p and q each independently represent an integer of 0 to 3, When p represents 2 or 3, each of the R³ may be the same or different and when q represents 2 or 3, each of the R⁴ may be the same or different.

3-Hydroxy-1-adamantyl (meth)acrylate and 3,5-dihydioxy-1-adamantyl (meth)acrylate can be produced, for example, by reacting corresponding hydroxyadamantane with (meth)acrylic acid or its acid halide, and they are also commercially available.

Further, (meth)acryloyloxy-γ-butyrolactone having a lactone ring optionally substituted by alkyl can be produced by reacting corresponding α- or β-bromo-γ-butyrolactone with acrylic acid or methacrylic acid, or reacting corresponding α- or β-hydroxy-γ-butyrolactone with acrylic halide or methacrylic halide.

As monomers to give structural units of the formulae (a) and (b), specifically listed are, for example, (meth)acrylates of alicyclic lactones having hydroxyl described below, and mixtures thereof, and the like. These esters can be produced, for example, by reacting corresponding alicyclic lactone having hydroxyl with (meth)acrylic acids, and the production method thereof is described in, for example, JP2000-26446-A

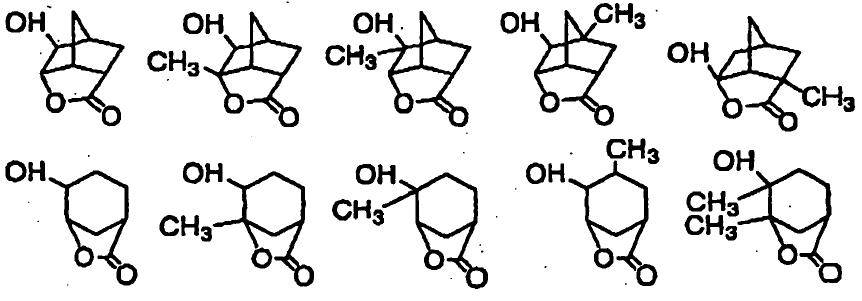

Examples of the (meth)acryloyloxy-γ-butyrolactone having a lactone ring optionally substituted by alkyl (e.g. 1 to 4 carbon atoms) include α-acryloyloxy-γ-butyrolactone, α-methacryloyloxy-γ-butyrolactone, α-acryloyloxy-β,β-dimethyl-γ-butyrolactone, α-methacryloyloxy-β,β-dimethyl-γ-butylactone, α-acryloyloxy-α-methyl-γ-butyrolactone, α-methacryloyloxy-α-methyl-γ-butyrolactone, β-acryloyloxy-γ-butyrolactone, β-methacryloyloxy-γ-butyrolactone, β-methacryloylaxy-α-methyl-γ-butyrolactone and the like.

In the case of KrF lithography, even in the case of using a structural unit derived from hydroxystyrene such as p- and m-hydroxystyrene, as one of the resin components, resist composition having sufficient transparency can be obtained. For obtaining such copolymerization resins, the corresponding (meth)acrylic ester monomer can be radical-polymerized with acetoxystyrene and styrene, and then the acetoxy group in the structural unit derived from acetoxystyrene can be de-acetylated with an acid.

The resin containing a structural unit derived from 2-norbomene shows strong structure because the alicyclic group is directly present on its main chain and has excellent dry etching resistance. The structural unit derived from 2-norbomene can be introduced into the main chain by radical polymerization using, for example, in addition to corresponding 2-norbornene, aliphatic unsaturated dicarboxylic anhydrides such as maleic anhydride and itaconic anhydride together. The structural unit derived from 2-norbomene is formed by opening of its double bond, and can be represented by the formula (c). The structural unit derived from maleic anhydride and the structural unit derived from itaconic anhydride which are the structural units derived from aliphatic unsaturated dicarboxylic anhydrides are formed by opening of their double bonds, and can be represented by the formula (d) and the formula (e), respectively.

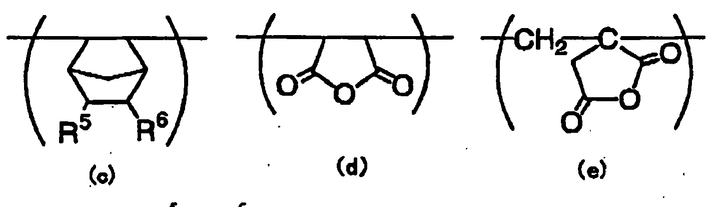

Here, R⁵ and R⁶ in the formula (c) each independently represent hydrogen, alkyl having 1 to 3 carbon atoms, hydroxyalkyl having 1 to 3 carbon atoms, carboxyl, cyano or -COOU group in which U represents alcohol residue, or R⁵ and R⁶ can bond together to form a carboxylic anhydride residue represented by -C(=O)OC(=O)-.

In R⁵ and R⁶, examples of the alkyl include methyl, ethyl, propyl and isopropyl, specific examples of hydroxyalkyl include hydroxymethyl, 2-hydroxyethy and the like.

In R⁵ and R⁶, -COOU group is an ester formed from carboxyl, and as the alcohol residue corresponding to U, for example, optionally substituted alkyls having about 1 to 8 carbon atoms, 2-oxooxolan-3- or -4-yl and the like are listed, and as the substituent on the alkyl, hydroxyl, alicyclic hydrocarbon residues and the like are listed.

Specific examples of the monomer used to give the structural unit represented by the formula (c) may include the followings;
2-norbomene,
2-hydroxy-5-norbomene,
5-norbomen-2-carboxylic acid,
methyl 5-norbomen-2-carboxylate,
2-hydroxyethyl 5-norbomen-2-carboxylate,
5-norbomen-2-methanol,
5-norbomen-2, 3-dicarboxylic acid anhydride, and the like.

When U in -COOU is acid-labile group, the structural unit of the formula (c) is a structural unit having acid-labile group even if it has norbornene structure. Examples of monomers giving structural unit having add-labile group include t-butyl 5-norbomen-2-carboxylate, 1-cyclohmyl-1-methylethyl 5-norbomen-2-carboxylate, 1-methylcyclohexyl 5-norbomen-2-carboxylate, 2-methyl-2-adamantyl 5-norbomen-2-carboxylate, 2-elhyl-2-adamantyl 5-norbomen-2-carboxylate, 1-(4-methylcyclohexyl)-1-methylethyl 5-norbomen-2-carboxylate, 1-(4-hydroxylcyclohexyl)-1-methylethyl 5-norbomen-2-carboxylate,1-methyl-1-(4-oxocyclohexyl)ethyl 5-norbomen-2-carboxylate, 1-(1-adamantyl)-1-methylethyl 5-norbomen-2-carboxylate, and the like.

The resin used in the present composition preferably contains structural unit(s) having an acid-labile group generally in a ratio of 10 to 80% by mol in all structural units of the resin though the ratio varies depending on the kind of radiation for patterning exposure, the kind of an acid-labile group, and the like.

When the structural units particularly derived from 2-alkyl-2-adamantyl (meth)acrylate or 1-(1-adamantyl)-1-alkylalkyl (meth)acrylate are used as the acid-labile group, it is advantageous that the ratio of the structural units is 15% by mol or more in all structural units of the resin.

When, in addition to structural units having an acid-labile group, other structural units having acid-stable group are contained, it is preferable that the sum of these structural units is in the range of 20 to 90% by mol based on all structural units of the resin.

When alicyclic compound having olefinic double bond and aliphatic unsaturated dicarboxylic anhydride are used as copolymerization monomers, it is preferable to use them in excess amount in view of a tendency that these are not easily polymerized.

In the present composition, performance deterioration caused by inactivation of acid which occurs due to post exposure delay can be diminished by adding basic compounds, particularly, basic nitrogen-containing organic compounds, for example, amines as a quencher.

Specific examples of such basic nitrogen-containing organic compounds include the ones represented by the following formulae:

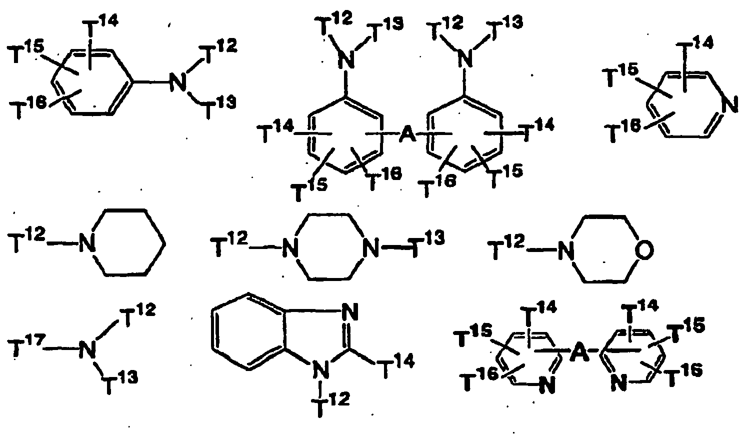

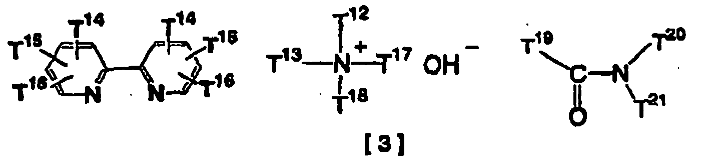

In the formulas, T¹² and T¹³ each independently represent a hydrogen atom, an alkyl group, a cycloalkyl group or an aryl group. The alkyl group preferably bas about 1 to 6 carbon atoms, the cycloalkyl group preferably has about 5 to 10 carbon atoms, and the aryl group preferably has about 6 to 10 carbon atoms. Furthermore, at least one hydrogen atom on the alkyl group, cycloalkyl group or aryl group may each independently be substituted by hydroxyl group, amino group, or alkoxy group having 1 to 6 carbon atoms. At least one hydrogen atom on the amino group may each independently be substituted by alkyl group having 1 to 4 carbon atoms.

T¹⁴, T¹⁵ and T¹⁶ each independently represent a hydrogen atom, an alkyl group, a cycloalkyl group, an aryl group or an alkoxy group. The alkyl group preferably has about 1 to 6 carbon atoms, the cycloalkyl group preferably has about 5 to 10 carbon atoms, the aryl group preferably has about 6 to 10 carbon atoms, and the alkoxy group preferably has about 1 to 6 carbon atoms. Furthermore, at least one hydrogen atom on the alkyl group, cycloalkyl group, aryl group or alkoxy group may each independently be substituted by hydroxyl group, amino group, or alkoxy group having 1 to 6 carbon atoms. At least one hydrogen atom on the amino group may be substituted by alkyl group having 1 to 4 carbon atoms.

T¹⁷ represents an alkyl group or a cycloalkyl group. The alkyl group preferably has about 1 to 6 carbon atoms, and the cycloalkyl group preferably has about 5 to 10 carbon atoms. Furthermore, at least one hydrogen atom on the alkyl group or cycloalkyl group may each independently be substituted by hydroxyl group, amino group, or alkoxy group having 1 to 6 carbon atoms. At least one hydrogen atom on the amino group may be substituted by alkyl group having 1 to 4 carbon atoms.

In the formulas, T¹⁸ represents an alkyl group, a cycloalkyl group or an aryl group. The alkyl group preferably has about 1 to 6 carbon atoms, the cycloalkyl group preferably has about 5 to 10 carbon atoms, and the aryl group preferably has about 6 to 10 carbon atoms. Furthermore, at least one hydrogen atom on the alkyl group, cycloalkyl group or aryl group may each independently be substituted by a hydroxyl group, an amino group, or an alkoxy group having 1 to 6 carbon atoms. At least one hydrogen atom on the amino group may each independently be substituted by alkyl group having 1 to 4 carbon atoms.

However, none of T¹² and T¹³ in the compound represented by the above formula [3] is a hydrogen atom.

A represents alkylene group, carbonyl group, imino group, sulfide group or disulfide group. The alkylene group preferably has about 2 to 6 carbon atoms.

Moreover, among T¹²-T¹⁸, in regard to those which can be straight-claimed or branched, either of these may be permitted.

T¹⁹, T²⁰ and T²¹ each independently represent a hydrogen atom, an alkyl group having 1 to 6 carbon atoms, an aminoalkyl group having 1 to 6 carbon atoms, a hydroxyalkyl group having 1 to 6 carbon atoms or a substituted or unsubstituted aryl group having 6 to 20 carbon atoms, or T¹⁹ and T²⁰ bond to form an alkylene group which forms a lactam ring together with adjacent -CO-N-.

Examples of such compounds includes hexylamine, heptylamine, octylamine, nonylamine, decylamine, aniline, 2-, 3- or 4-methylaniline, ethylenediamine, tetramethylenediamine, hexamethylenediamine, 4,4'-diamino-1,2-diphenylethane, 4,4'-diamino-3,3'-dimethyldiphenylmethane, 4,4'-diamino-3,3'-diethyldiphenylmethane, dibutylamine, dipentylamine, dihexylamine, diheptylamine, dioctylamine, dinonylamine didecylamine, N-methylaniline, piperidine, diphenylamine, triethylamine, trimethylamine, tripropylamine, tributylamine, tripentylamine, trihexylamine, triheptylamine, trioctylamine, trinonylamine, tridecylamine, methyldibutylamine, methyldipentylamine, methyldihexylamine, methyldicyclohexylamine, methyldiheptylamine, methyldioctylamine, methyldinonylamine, methyldidecylamine, ethyldibutylamine, ethyldipentylamine, ethyldihexylamine, ethyldiheptylamine, ethyldioctylamine, ethyldinonylamine, ethyldidecylamine, dicyclohexylmethylamine, tris[2-(2-methoxyethoxy)ethyl]amine, triisopropanolamine, N,N-dimethylaniline, 2,6-isopropylaniline, pyridine, 4-methylpyridine, bipyridine, 2,2'-dipyridylamine, di-2-pyridyl ketone, 1,2-di(2-pyridyl)ethane, 1,2-di(4-pyridyl)ethane, 1,3-di(4-pyridyl)propane, 1,2-bis(2-pyridyl)ethylene, 1,2-bis(4-pyridyl)ethylene, 4,4'-dipyridyl sulfide, 4,4'-dipyridyl disulfide, 1,2-bis(4-pyridyl)ethylene, 2,2'-dipicolylamine, 3,3'-dipicolylamine, tetramethylammonium hydroxide, tetraisopropylammonium hydroxide, tetrabutylammonium hydroxide, tetra-n-hexylammonium hydroxide, tetra-n-octylammonium hydroxide, phenyltrimethylammonium hydroxide, 3-trifluoromethylphenyltrimethylammonium hydroxide, (2-hydroxyethyl)trimethylammonium hydroxide (so-called "choline"), N-methylpyrrolidone, and the like.

Furthermore, hindered amine compounds having piperidine skeleton as disclosed in JP-A-H11-52575 can be used as quencher.

It is preferable that the present composition contains resin component in an amount of about 80 to 99.9% by weight and Salt (L) in an amount of 0.1 to 20% by weight on the total amount of the resin component and Salt (L).

When basic compound is used as a quencher, the basic compound is contained preferably in an amount of about 0.01 to 1 part by weight per 100 parts by weight of sum of resin component and Salt (L).

The present composition can contain, if necessary, various additives in small amounts such as a sensitizer, solution suppressing agent, other polymers, surfactant, stabilizer, dye and the like, as long as the effect of the present invention is not prevented.

The present composition is usually in the form of a resist liquid composition in which the aforementioned ingredients are dissolved in a solvent, and the resist liquid composition is to be applied onto a substrate such as a silicon wafer by a conventional process such as spin coaling. The solvent used here is sufficient to dissolve the aforementioned ingredients, have an adequate drying rate, and give a uniform and smooth coat after evaporation of the solvent and, hence, solvents generally used in the art can be used. In the present invention, the total solid content means total content exclusive of solvents.

Examples thereof include glycol enter esters such as ethyl Cellosolve acetate, methyl Cellosolve acetate and propylene glycol monomethyl ether acetate; glycol ethers such as propylene glycol monomethyl ether, di(ethylene glycol) dimethyl ether, esters such as ethyl lactate, butyl lactate, amyl lactate and ethyl pyruvate and the like; ketones such as acetone, methyl isobutyl ketone, 2-heptanone and cyclohexanone; cyclic esters such as γ-butyrolactone, and the like. These solvents can be used each alone or in combination of two or more.

A resist film applied onto the substrate and then dried is subjected to exposure for patterning, then heat-treated for facilitating a deblocking reaction, and thereafter developed with an alkali developer. The alkali developer used here may be any one of various alkaline aqueous solutions used in the art, and generally, an aqueous solution of tetramethylammonium hydroxide or (2-hydroxyethyl)trimethylammonium hydroxide (commonly known as "choline") is often used.

It should be construed that embodiments disclosed here are examples in all aspects and not restrictive. It is that the scope of the invention is determined not by the above descriptions but by appended Claims, and includes all variations of the equivalent meanings and ranges to the Claims.

The present invention will be described more specifically by way of examples, which are not construed to limit the scope of the present invention. The "%" and "part(s)" used to represent the content of any component and the amount of any material used in the following examples and comparative examples are on a weight basis unless otherwise specifically noted. The weight-average molecular weight of any material used in the following examples is a value found by gel permeation chromatography [HLC-8120GPC Type, Column (Three Columns): TSKgel Multipore HXL-M, Solvent: Tetrahydrofuran, manufactured by TOSOH CORPORATION] using styrene as a standard reference material. Structures of compounds were determined by NMR (GX-270 Type, or EX-270 Type, manufactured by JEOLLTD) and mass spectrometry (Liquid Chromatography : 1100 Type, manufactured by AGILENT TECHNOLOGIES LTD, Mass Spectrometry: LC/MSD Type or LC/MSD TOF Type, manufactured by AGILENT TECHNOLOGIES LTD.).

### Synthetic Example 1

### Synthesis of triphenylsulfonium 4-oxo-1-adamentyloxycarbonyldifluoromethanesulfonate

(1) 230 Parts of 30% aqueous sodium hydroxide solution were added into a mixture of 100 parts of methyl difluoro(fluorosulfonyl)acelate and 250 parts of ion-exchanged water in an ice bath. The added mixture was heated and refluxed at 100°C for 3 hours. After cooling, the cooled mixture was neutralized with 88 parts of conc. hydrochloric acid and concentrated to obtain 164,8 parts of sodium difluorosulfoacetate (containing inorganic salt, purity: 62.6%).
(2) 5.0 Parts of sodium difluorosulfoacetate (purity: 62.8%), 2.6 parts of 4-oxo-1-adamantanol and 100 parts of ethylbenzene were charged in a vessel, 0.8 parts of cone. Sulfuric acid were added thereto, and the mixture was refluxed for 42 hours. After cooling, the resultant mixture was filtrated to obtain crystals, and the crystals were washed with tert-butyl methyl ether to obtain 5.5 parts of sodium salt of 4-oxo-1-adamantyl difluorosulfoacetate. The purity thereof was 35.6%, which was determined by ¹H-NMR. 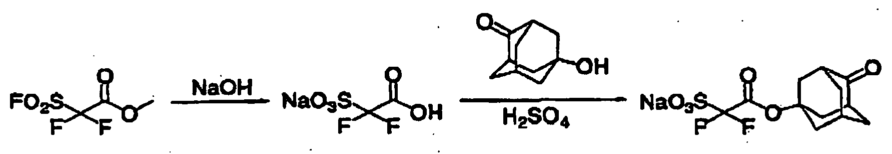 ¹H-NMR(dimethylsulfoxide-d₆, Internal Standard; tetramethylsilane):d(ppm) 1.84 (d, 2H, J=13.0Hz); 2.00 (d, 2H, J=11.9Hz); 229-2.32 (m, 7H); 254(s, 2H)
(3) 5.4 Parts of sodium salt of 4-oxo-1-adamantyl difluorosulfoacetate obtained in (2) (purity: 35.6%) were charged in a vessel, and a mixed solvent of 16 parts of acetonitrile and 16 parts of ion-exchanged water was added thereto to give a mixture. A solution prepared by mixing 1,7 parts of triphenylsulfonium chloride, 5 parts of acetonitrile and 5 parts of ion-exchanged water was added to the mixture. After stirring for 15 hours, the stirred mixture was concentrated and extracted with 142 parts of chloroform. The organic layer was washed with ion-exchanged water. The obtained organic layer was concentrated. The concentrate was washed with 24 parts of tert-butyl methyl ether and the solvent was decanted and the crystals were dried to obtain 1.7 parts of triphenylsulfonium 4-oxo-1-adamantyloxycarbonyldifluoromethanesulfonate, which is called acid generator B1. 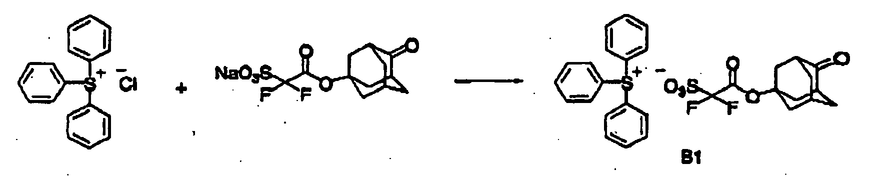 ¹H-NMR(dimethylsulfoxide-d₆, Internal Standard: tetramethylsilane):d(ppm) 1.83 (d, 2H, J=12.7Hz); 2.00 (d, 2H, J=12.0Hz); 229-232 (m, 7H); 253 (s, 2H); 7.75-7.91 (m, 15H)
   MS (EST(+)Spectrum):M+263.2 (C₁₈H₁₅S⁺ =263.09)
   MS (ESI(-) Spectrum): M-323.0 (C₁₂H₁₃F₂O₆S*⁻=*323.04)

### Synthetic Example 2

### Synthesis of triphenylsulfonium 4-hydroxy-1-adamantyloxycarbonyldifluoromethanesulfoante

(1) 4.69 Parts of triphenylsulfonium 4-oxo-1-adamantyloxycarbonyldifluoromethanesulfonate were dissolved in 15 parts of acetonitrile. Into the solution was added dropwise a solution obtained by dissolving 0.15 parts of sodium borohydride in 151 parts of ion-exchanged water. After the added mixture was stirred for 5 hours, 1N hydrochloric acid was added thereto, and extracted with 113 parts of chloroform. After repeatedly washing with ion-exchanged water, the organic layer obtained was concentrated. To the concentrate, acetonitrile was added, then the added mixture was concentrated. The concentrate was added dropwise into ethyl acetate. The mixture was filtrated to obtain solid. The solid was dried to obtain 220 parts of triphenylsulfonium 4-hydroxy-1-adamantyloxycarbonyldifluoromethaneuslfonate in the form of white solid, which is called acid generator B2 As a result of analyses by ¹H-NMR and LCMS, the acid generator B2 was determined to be a mixture of two isomers. 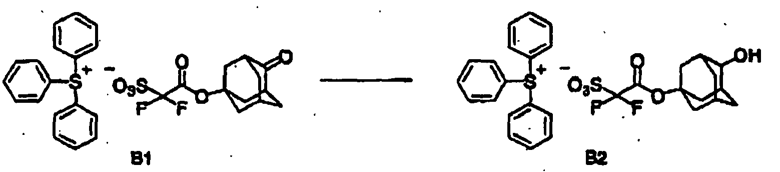 ¹H-NMR spectram thereof is shown in Figure 1.
   MS (ESI(+) Spectrum): M+ 263.0 (C₁₈H₁₅ S⁺=263.09)
   MS (ESI(-)Spectrum):M-325.0 (C₁₂H₁₃F₂O₆S⁻=325.06)

### Synthetic Example 3

### Synthesis of 1-(2-oxo-2-phenylethyl)tetrahydrothiophenium 4-oxo-1-adamantyloxycarbonyldifluoromethanesulfonate.

10 Parts of sodium salt of 4-oxo-1-adamantyl difluorosulfoacetate (purity: 55.2%) were charged in a vessel, and a mixed solvent of 30 parts of acetonitrile and 20 parts of ion-exchanged water was added thereto to give a mixture. A solution prepared by mixing 5.0 parts of 1-(2-oxo-2-phenylethyl)tetrahydrothiophenium bromide, 10 parts of acetonitrile and 20 parts of ion-exchanged water was added to the mixture. After stirring for 15 hours, the stirred mixture was concentrated and extracted with 98 parts of chloroform. The organic layer was washed with ion-achanged water. The obtained organic layer was concentrated. The concentrate was washed with 70 parts of ethyl acetate and the solvent was decanted and the crystals were dri ed to obtain 5.2 parts of 1-(2-oxo-2-phenylethyl)tetrahydrothiophenium 4-oxo-1-adamantyloxycarbonyldifluoromethanesulfonate in the form of white solid, which is called acid generator B3. 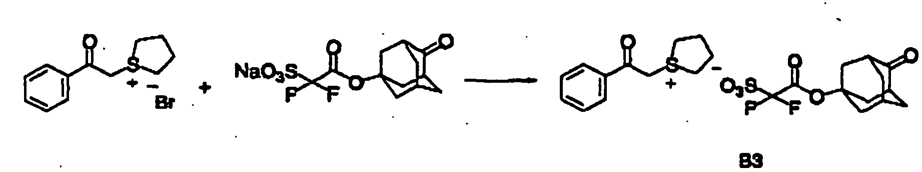 ¹H-NMR(dimethylsulfoxide-d₆, Internal Standard: Tetramethylsilane):
δ (ppm) 1.83 (d, 2H, J=12.5Hz); 2.00 (d, 2H, J=12.0Hz); 2212.37 (m, 11H); 2.53 (s, 2H); 3.47-3.62 (m, 4H); 531 (s, 2H); 7.63 (t, 2H, J=7.3Hz); 7.78 (t, 1H, J=7.6Hz); 8.01 (dd, 2H, J=1.5Hz, 73Hz)
MS (ESI(+) Spectrum): M+ 207.1 (C₁₂H₁₅ OS⁺ =207.08)
MS (ESI(-) Spectrum): M- 323.0 (C₁₂ H₁₃ F₂ O₆ S⁻ =323.04)

### Referential Synthetic Example 1

### Synthesis of triphenylsulfonium 1-adamantylmethoxycarbonyldifluoromethanesulfonate

(1) 230 Parts of 30% aqueous sodium hydroxide solution were added into a mixture of 100 parts of methyl difluoro(fluorosulfonyl)acetate and 250 parts of ion-exchanged water in an ice bath. The added mixture was heated and refluxed at 100°C for 3 hours. After cooling, the cooled mixture was neutralized with 88 parts of cone. hydrochloric acid and concentrated to obtain 164.8 parts of sodium difluorosulfoacetate (containing inorganic salt, purity: 62.6%).
(2) 39.4 Parts of sodium difluorosulfoacetate (purity: 63.5%), 21.0 parts of 1-adamantanemethanol and 200 parts of dichloroethane were charged in a vessel, 24.0 parts of p-toluenesulfonic acid were added thereto, and the mixture was refluxed for 7 hours, After concentrating the mixture to eliminate dichloroethane, 250 parts of tert-butyl methyl ether were added thereto, and the added mixture was stirred. The stirred mixture was filtered to obtain solid 250 parts of acetonitrile were added to the solid. The mixture was stirred and filtrated. The filtrate was concentrated to obtain 32.8 parts of sodium salt of 1-adamantylmethyl difluorosulfoacetate. 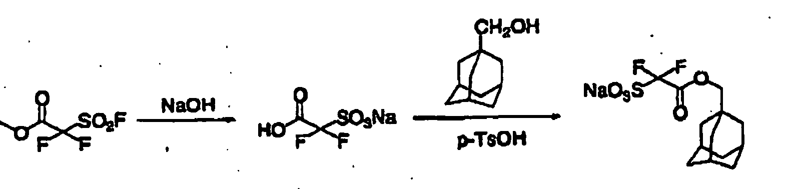
(3) 32.8 parts of sodium salt of 1-adamantymethyl difluorosulfoacetate obtained in (2) were dissolved in 100 parts of ion-exchanged water. 28.3 Parts of triphenylsulfonium chloride and 140 parts of methanol were added to the solution. After stirring for 15 hours, the stirred mixture was concentrated and extracted with 200 parts of chloroform twice. The organic layers were mixed and washed with ion-exchanged water. The obtained organic layer was concentrated. 300 Parts of tert-butyl methyl ether were added to the concentrate, the mixture was stirred and filtered to obtain solid. The solid was dried under reduced pressure to obtain 39.7 parts of triphenylsulfonium 1-adamantylmethyldifluoromethanesulfonate in the form of white crystals, which is called acid generator C1. 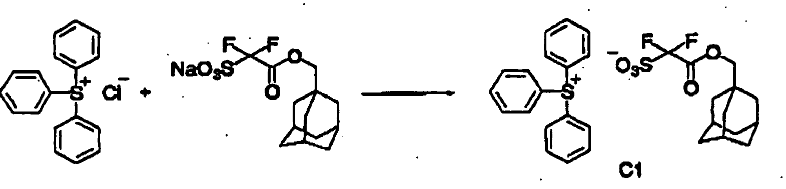 ¹H-NMR (dimethylsufloxide-d₆, Internal Standard: tetramethylsilane): d(ppm) 1-52(d, 6H); 1.63(dd, 6H); 1.93(s, 3H); 3.81(s, 2H); 7.76-7-90(m, 15H)
   MS (ESI(+) Spectrum): M+ 263.2 (C₁₈H₁₅, S⁺ = 263.09)
   MS(ESI(-)Spectrum):M-323.0(C₁₃H₁₇F₂O₅S = 323.08)

### Resin synthesis Example 1 Synthesis of resin A1

2-Ethyl-2-adamantyl methacrylate, 3-hydroxy-1-adamantyl methacrylate and α-methacryloyloxy-γ-butyrolactone were charged at a molar ratio of 5:2.5:2.5 (20.0 parts:9.5 parts:7.3 parts), and methyl isobutyl ketone in twice the weight based on all monomers was added, to prepare solution. To the solution was added azobisisobutyronitrile as an initiator in a ratio of 2 mol% based on all monomer molar amount, and the mixture was heated at 80°C for about 8 hours. Then, the reaction solution was poured : into a large amount of heptane to cause precipitation, and this operation was repeated three times for purification. As a result, copolymer having a weight-average molecular weight of about 9,200 was obtained This is called resin A1.

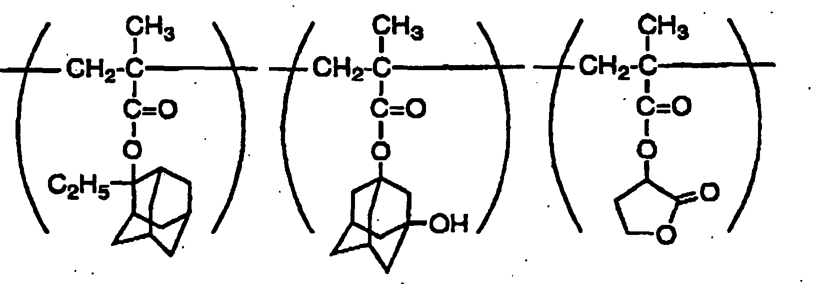

### Examples 1 to 2 Comparative Examples 1 to 2

The following components were mixed and dissolved and further filtrated through a fluorine resin filter having pore diameter of 0.2 µm to prepare resist liquid.
Resin
resin A1 : 10 parts
Acid generator
acid generator B1: 0.25 parts 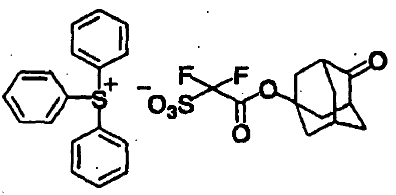 acid generator B2:0.26 parts 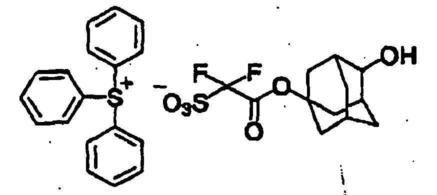 acid generator C1: 0.26 parts 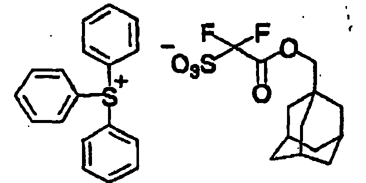 acid generator C2: triphenyl sulfonium perfluorobutanesulfonate 0.25 parts
Kind used in Examples is described in Table 1
Quencher
quencher Q1: 2,6-diisopropylaniline 0.0325 parts
Solvent

| | | |
|---|---|---|
| Solvent Y1: | propylene glycol monomethyl ether acetate | 51.5 parts |
| | 2-heptanone | 35.0 parts |
| | γ-butyrolactone | 3.5 parts |

Silicon wafers were each coated with "ARC-29A-8", which is an organic anti-reflective coating composition available from Brewer Co., and then baked under the conditions: 215°C, 60 seconds, to form a 78OÅ-thick organic anti-reflective coating. Each of the resist liquids prepared as above was spin-coated over the anti-reflective coating so that the thickness of the resulting film became 0.25µm after drying. The silicon wafers thus coated with the respective resit liquids were each prebaked on a direct hotplate at at temperature of 130°C for 60 seconds. Using an ArF excimer stepper ("NSR ArF" manufactured by Nikon Corporation, NA=0.55,2/3 Annular) each wafer thus formed with the respective resist film was subjected to line and space pattern exposure with the exposure quantity being varied stepwise.

After the exposure, each wafer was subjected to post-exposure baking on a hotplate at a temperature of 130°C for 60 seconds and then to paddle development for 60 seconds with an aqueous solution of 2.38wt% tetramethylammonium hydroxide.

Each of a dark field pattern developed on the organic anti-reflective coating substrate after the development was observed with a scanning electron microscope, the results of which are shown in Table 1. The term "dark field pattern", as used herein, means a pattern obtained by exposure and development through a reticle comprising chromium base surface (light-shielding portion) and linear glass layers (light-transmitting portion) formed in the chromium surface and aligned with each other. Thus, the dark field pattern is such that, after exposure and development, resist layer surrounding the line and space pattern remains on substrate.

### Resolution:

It is expressed as the minimum size of space pattern which gave the space pattern split by the line pattern at the exposure amount of the effective sensitivity. Herein, the effective sensitivity is expressed by the amount of exposure that the line pattern (light-shielding layer) and the space pattern (light-transmitting layer) become 1:1 after exposure through 0.13µm line and space pattern mask and development.
Profile T/B

It is expressed by a ratio of top side length (referred to as T) and bottom side length (referred to as B) in line part of 0.13µm line and space pattern. The closer to 1 the ratio is, the better the profile of its resist pattern is.

**Table 1**

| No. | Acid Generator | Resolution (µm) | Profile T/B |
|---|---|---|---|
| Example 1 | B1 | 0.12 | 1.00 |
| Example 2 | B2 | 0.12 | 1.04 |
| Comparative Example 1 | C1 | 0.13 | 0.86 |
| Comparative Example 2 | C2 | 0.13 | 0.71 |

As is apparent from Table 1, the resist compositions of the Examples, which accord to the present invention, give better resist patterns in resolution and in pattern profile than those of the Comparative Examples.

Salt (L) is suitably useful for an add generator for chemically amplified positive least composition. The present composition provides excellent resist patterns in resolution and pattern profile and is especially suitable for ArF excimer laser lithography, KrF excimer laser lithography and ArF immersion lithography.

## Claims

1. A salt of the formula (L): 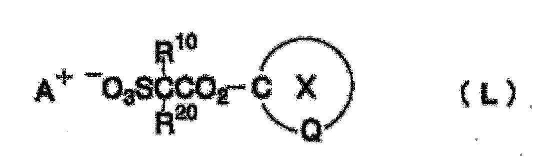 wherein Q represents -CO- group or -C(OH)- group; ring X represents monocyclic or polycyclic hydrocarbon group having 3 to 30 carbon atoms in which a hydrogen atom is substituted by a hydroxyl group at Q position when Q is -C(OH)- group or in which two hydrogen atoms are substituted by =O group at Q position when Q is -CO- group, and at least one hydrogen atom in the monocyclic or polycyclic hydrocarbon group may optionally be substituted by an alkyl group having 1 to 6 carbon atoms, alkoxy group having 1 to 6 carbon atoms, perfluoroalkyl group having 1 to 4 carbon atoms, hydroxyalkyl group having 1 to 6 carbon atoms, hydroxyl group or cyano group; R¹⁰ and R²⁰ each independently represent fluorine atom or perfluoroalkyl group having 1 to 6 carbon atoms; and A⁺ represents organic counter ion.

2. A salt of the formula (LI) 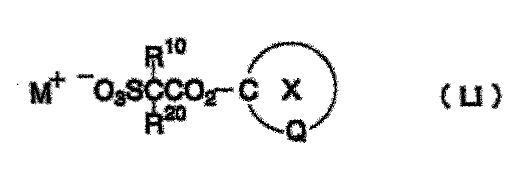 wherein Q represents -CO- group or -C(OH)- group; X represents monocyclic or polycyclic hydrocarbon group having 3 to 30 carbon atoms in which a hydrogen atom is substituted by a hydroxyl group at Q position when Q is -C(OH)- group or in which two hydrogen atoms are substituted by =O group at Q position when Q is -CO- group, and at least one hydrogen atom in the monocyclic or polycyclic hydrocarbon group may optionally be substituted by an alkyl group having 1 to 6 carbon atoms, alkoxy group having 1 to 6 carbon atoms, perfluoroalkyl group having 1 to 4 carbon atoms, hydroxyalkyl group having 1 to 6 carbon atoms, hydroxyl group or cyano group; R¹⁰ and R²⁰ each independently represent fluorine atom or perfluoroalkyl group having 1 to 6 carbon atoms; and M represents Li, Na, K or Ag.

3. The salt according to Claim 2, wherein the salt has the formula (V) 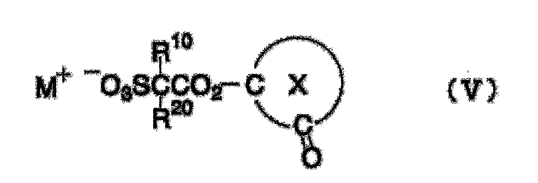 wherein X, R¹⁰, R²⁰ and M have the same meanings as defined in Claim 2.

4. The salt according to Claim 2, wherein the salt has the formula (VI) 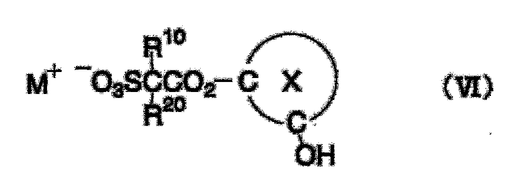 wherein X, R¹⁰, R²⁰ and M have the same meanings as defined in Claim 2.

5. A process for producing a salt of the formula (LI) 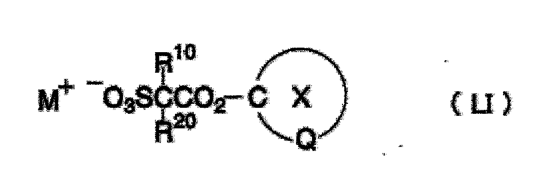 wherein Q represents -CO- group or -C(OH)- group; X represents monocyclic or polycyclic hydrocarbon group having 3 to 30 carbon atoms in which a hydrogen atom is substituted by a hydroxyl group at Q position when Q is -C(OH)- group or in which two hydrogen atoms are substituted by =O group at Q position when Q is -CO- group, and at least one hydrogen atom in the monocyclic or polycyclic hydrocarbon group may optionally be substituted by an alkyl group having 1 to 6 carbon atoms, alkoxy group having 1 to 6 carbon atoms, perfluoroalkyl group having 1 to 4 carbon atoms, hydroxyalkyl group having 1 to 6 carbon atoms, hydroxyl group or cyano group; R¹⁰ and R²⁰ each independently represent fluorine atom or perfluoroalkyl group having 1 to 6 carbon atoms; and M represents Li, Na, K or Ag, which comprises esterifying an alcohol of the formula (LII) 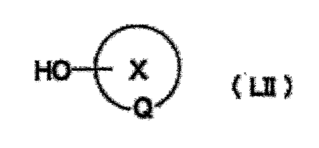 wherein X and Q have the same meanings as defined above,
with a carboxylic acid of the formula (IX) 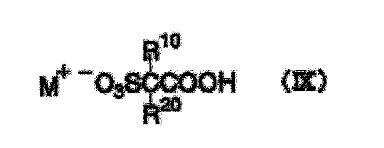 wherein R¹⁰, R²⁰ and M have the same meanings as defined above.

6. A process for producing a salt of the formula (II) 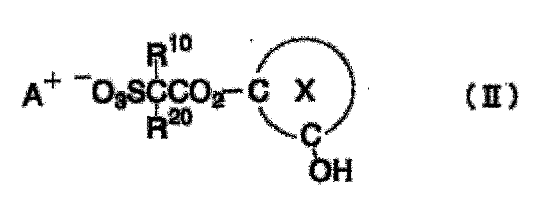 wherein ring X represents monocyclic or polycyclic hydrocarbon group having 3 to 30 carbon atoms in which a hydrogen atom is substituted by a hydroxyl group at Q position when Q is -C(OH)- group or in which two hydrogen atoms are substituted by =O group at Q position when Q is -CO- group, and at least one hydrogen atom in the monocyclic or polycyclic hydrocarbon group may optionally be substituted by an alkyl group having 1 to 6 carbon atoms, alkoxy group having 1 to 6 carbon atom, perfluoroalkyl group having 1 to 4 carbon atoms, hydroxyalkyl group having 1 to 6 carbon atoms, hydroxyl group or cyano group; R¹⁰ and R²⁰ each independently represent fluorine atom or perfluoroalkyl group having 1 to 6 carbon atoms; and A⁺ represents organic counter ion,
which comprises reducing a salt of the formula (I) 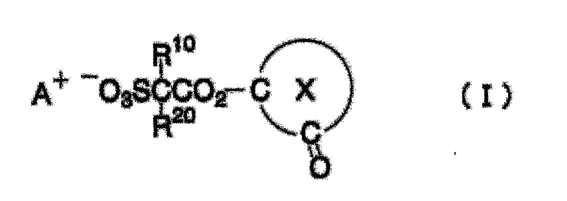 wherein X, A, R¹⁰ and R²⁰ have the same meanings as defined above.

7. A process for producing a salt of the formula (VI) 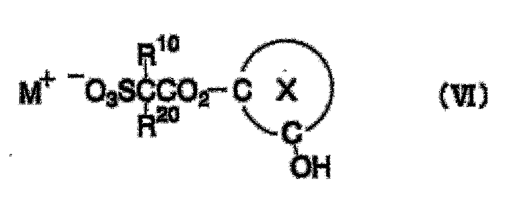 wherein X represents monocyclic or polycyclic hydrocarbon group having 3 to 30 carbon atoms in which a hydrogen atom is substituted by a hydroxyl group at Q position when Q is -C(OH)- group or in which two hydrogen atoms are substituted by =O group at Q position when Q is -CO- group, and at least one hydrogen atom in the monocyclic or polycyclic hydrocarbon group may optionally be substituted by an alkyl group having 1 to 6 carbon atoms, alkoxy group having 1 to 6 carbon atoms, perfluoroalkyl group having 1 to 4 carbon atoms, hydroxyalkyl group having 1 to 6 carbon atoms, hydroxyl group or cyano group; R¹⁰ and R²⁰ each independently represent fluorine atom or perfluoroalkyl group having 1 to 6 carbon atoms; and M represents Li, Na, K or Ag,
which comprises reducing a salt of the formula (V) 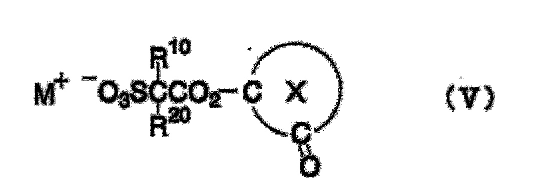 wherein X, R¹⁰, R²⁰ and M have the same meanings as defined above.

8. A process for producing a salt of the formula (LI) 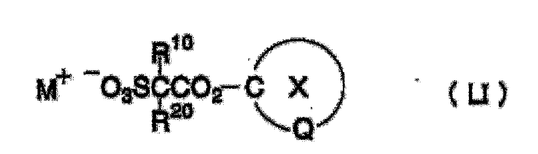 wherein Q represents -CO- group or -C(OH)- group; X represents monocyclic or polycyclic hydrocarbon group having 3 to 30 carbon atoms in which a hydrogen atom is substituted by a hydroxyl group at Q position when Q is -C(OH)- group or in which two hydrogen atoms are substituted by =O group at Q position when Q is -CO- group, and at least one hydrogen atom in the monocyclic or polycyclic hydrocarbon group may optionally be substituted by an alkyl group having 1 to 6 carbon atoms, alkoxy group having 1 to 6 carbon atoms, perfluoroalkyl group having 1 to 4 carbon atoms, hydroxyalkyl group having 1 to 6 carbon atoms, hydroxyl group or cyano group; R¹⁰ and R²⁰ each independently represent fluorine atom or perfluoroalkyl group having 1 to 6 carbon atoms; and M represents Li, Na, K or Ag,
which comprises esterifying an alcohol of the formula (LII) 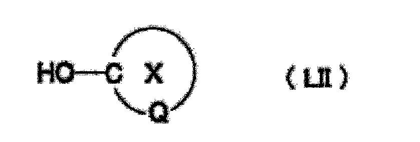 wherein X and Q have the same meaning as defined above,
with a carboxylic acid of the formula (X) 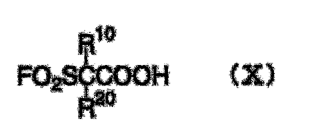 wherein R¹⁰ and R²⁰ have the same meanings as defined above,
and hydrolyzing with MOH, wherein M represents Li, Na, K or Ag.

9. A process for producing a salt of the formula (L) 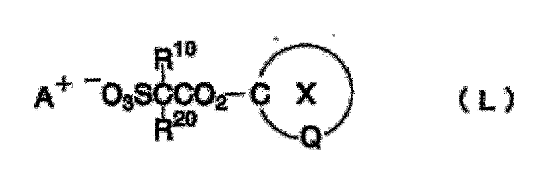 wherein Q represents -CO- group or -C(OH)- group; ring X represents monocyclic or polycyclic hydrocarbon group having 3 to 30 carbon atoms in which a hydrogen atom is substituted by a hydroxyl group at Q position when Q is -C(OH)- group or in which two hydrogen atoms are substituted by =O group at Q position when Q is -CO- group, and at least one hydrogen atom in the monocyclic or polycyclic hydrocarbon group may optionally be substituted by an alkyl group having 1 to 6 carbon atoms, alkoxy group having 1 to 6 carbon atoms, perfluoroalkyl group having 1 to 4 carbon atoms, hydroxyalkyl group having 1 to 6 carbon atoms, hydroxyl group or cyano group; R¹⁰ and R²⁰ each independently represent fluorine atom or perfluoroalkyl group having 1 to 6 carbon atoms; and A⁺ represents organic counter ion,
which comprises reacting a salt of the formula (LI) 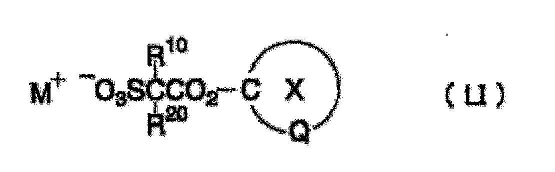 wherein M represents Li, Na, K or Ag, and Q, R¹⁰, R²⁰ and X have the same meanings as defined above,
with a compound of the formula (XI)
**A⁺ Z⁻** (XI)
wherein Z represents F, Cl, Br, I, BF₄, AsF₆, SbF₆, PF₆ or ClO₄ and A⁺ has the same meaning as defined above.

10. A chemically amplified resist composition comprising a salt of the formula (L) 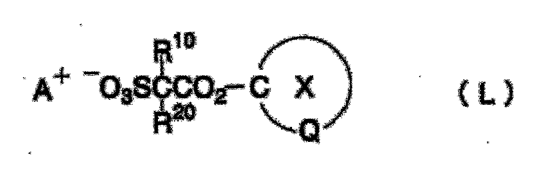 wherein Q represents -CO- group or -C(OH)- group; ring X represents monocylic or polycyclic hydrocarbon group having 3 to 30 carbon atoms in which a hydrogen atom is substituted by a hydroxyl group at Q position when Q is -C(OH)- group or in which two hydrogen atoms are substituted by =O group at Q position when Q is -CO- group, and at least one hydrogen atom in the monocyclic or polycyclic hydrocarbon group may optionally be substituted by an alkyl group having 1 to 6 carbon atoms, alkoxy group having 1 to 6 carbon atoms, perfluoroalkyl group having 1 to 4 carbon atoms, hydroxyalkyl group having 1 to 6 carbon atoms, hydroxyl group or cyano group; R¹⁰ and R²⁰ each independently represent fluorine atom or perfluoroalkyl group having 1 to 6 carbon atoms; and A⁺ represents organic counter ion, and
a resin which contains a structural unit having an acid-labile group and which resin is insoluble or poorly soluble in an alkali aqueous solution but becomes soluble in an alkali aqueous solution by the action of an acid.

11. The composition according to Claim 10, wherein the resin contains a structural unit derived from a monomer having a bulky and acid-labile group.

12. The composition according to Claim 11, wherein the bulky and acid-labile group is 2-alkyl-2-adamantyl group or 1-(1-adamantyl)-1-alkylalkyl group.

13. The composition according to Claim 11, wherein the monomer having bulky and acid-labile group is 2-alkyl-2-adamantyl (meth)acrylate, 1-(1-adamantyl)-1-alkylalkyl (meth)acrylate, 2-alkyl-2-adamantyl 5-norbornene-2-carboxylate, 1-(1-adamantyl)-1-alkylalkyl 5-norbornene-2-carboxylate, 2-alkyl-2-adamantyl α-chloroacrylate or 1-(1-adamantyl)-1-alkylalkyl α-chloroacrylate.

14. The composition according to any of Claims 10 to 13, wherein the composition further comprises a basic compound.

15. The salt, the process or the composition according to any of Claims 1 to 14, wherein each of R¹⁰ and R²⁰ is fluorine atom or trifluoromethyl group.

16. The salt, the process or the composition according to any of Claims 1, 10, 11, 12, 13, 14 and 15, wherein the salt has the following formula (I) 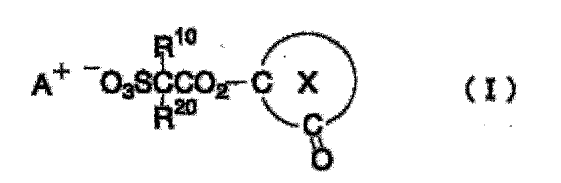 wherein X, A, R¹⁰ and R²⁰ have the same meanings as defined in Claim 10.

17. The salt, the process or the composition according to any of Claims 1, 10, 11, 12, 13, 14 and 15, wherein the salt has the following formula (II) 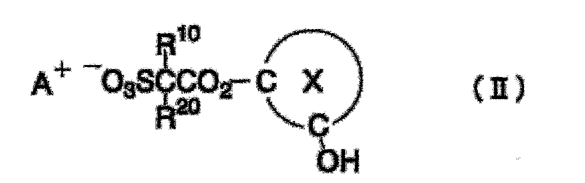 wherein X, A, R¹⁰ and R²⁰ have the same meanings as defined in Claim 10.

18. The salt, the process or the composition according to any of Claims 1, 10, 11, 12, 13, 14, 15, 16 and 17, wherein A⁺ is at least one cation selected from the group consisting of the formula (IIIa), the formula (IIIb), the formula (IIIc) and the formula (IIId):
a cation of the formula (IIIa) 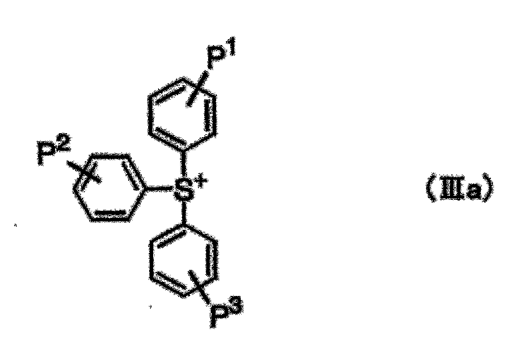
wherein P¹, P² and P³ each independently represent hydrogen atom, hydroxyl group, alkyl group having 1 to 12 carbon atoms or alkoxy group having 1 to 12 carbon atoms;
a cation of the formula (IIIb) 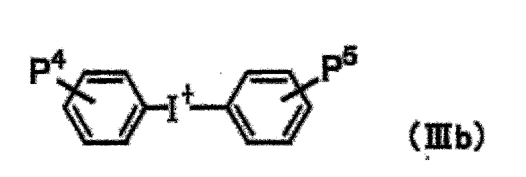 wherein P⁴ and P⁵ each independently represent hydrogen atom, hydroxyl group, alkyl group having 1 to 12 carbon atoms or alkoxy group having 1 to 12 carbon atoms;
a cation of the Formula (IIIc) 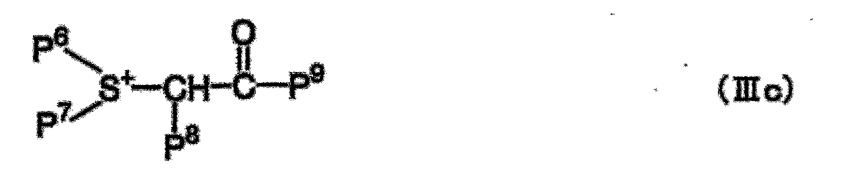 wherein P⁶ and P⁷ each independently represent alkyl having 1 to 12 carbon atoms or cycloalkyl having 3 to 12 carbon atoms, or P⁶ and P⁷ bond to form divalent acyclic hydrocarbon group having 3 to 12 carbon atoms which forms a ring together with the adj acent S⁺, and at least one -CH₂- in the divalent acyclic hydrocarbon group is optionally substituted by -CO-, -O- or -S-, P⁸ represents hydrogen, P⁹ represents alkyl having 1 to 12 carbon atoms, cycloalkyl having 3 to 12 carbon atoms or aromatic ring group optionally substituted, or P⁸ and P⁹ bond to form divalent acyclic hydrocarbon group which forms 2-oxocycloalkyl together with the adjacent -CHCO-, and at least one -CH₂- in the divalent acyclic hydrocarbon group is optionally substituted by -CO-, -O- or -S-, and
a cation of the formula (IIId) 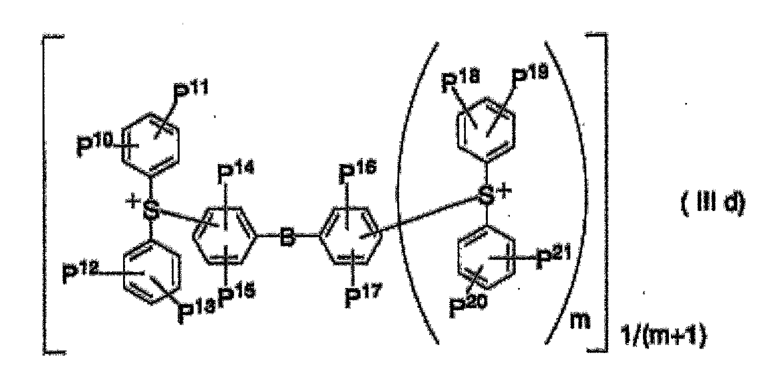 wherein P¹⁰, P¹¹, P¹², P¹³, P¹⁴, P¹⁵, P¹⁶, P¹⁷, P¹⁸, P¹⁹, P²⁰ and P²¹ each independently represent hydrogen atom, hydroxyl group, alkyl group having 1 to 12 carbon atoms or alkoxy group having 1 to 12 carbon atoms, B represents sulfur atom or oxygen atom, and m represents 0 or 1.

19. The salt, the process or the composition according to Claim 18, wherein A⁺ is a counter ion of the formula (IIIe): 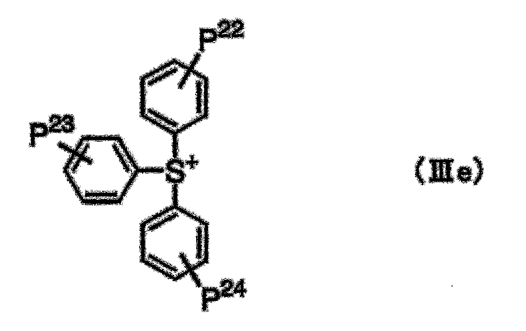 wherein P²² , P²³ and P²⁴ each independently represent hydrogen atom or alkyl group having 1 to 4 atoms.

20. The salt, the process or the composition according to any of Claims 1, 10, 11, 12, 13, 14, 15, 16, 17,18 and 19, wherein the ring X is cycloalkyl group having 4 to 8 carbon atoms, adamantyl group or norbornyl group, provided that, in each of the groups, a hydrogen atom is substituted by -OH group at Q position when Q is -C(OH)- group and two hydrogen atoms are substituted by =O at Q position when Q is -CO- group, and provided that at least one hydrogen atom in each of the groups may optionally be substituted by an alkyl group having 1 to 6 carbon atoms, alkoxy group having 1 to 6 carbon atoms, perfluoroalkyl group having 1 to 4 carbon atoms, hydroxyalkyl group having 1 to 6 carbon atoms, hydroxyl group or cyano group.

21. The salt or the composition according to any of Claims 1, 10, 11, 12, 13 and 14,
wherein the salt is one of the formula (IVa) 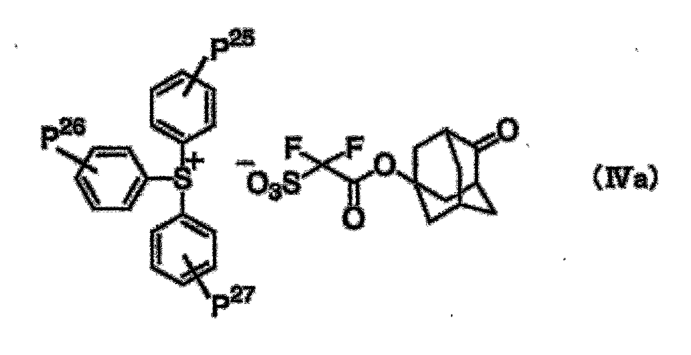 wherein P²⁵, P²⁶ and P²⁷ each independently represent hydrogen atoms or alkyl group having 1 to 4 carbon atoms.

22. The salt or the composition according to any of Claims 1, 10, 11, 12, 13 and 14,
wherein the salt is one of the formula (IVb)

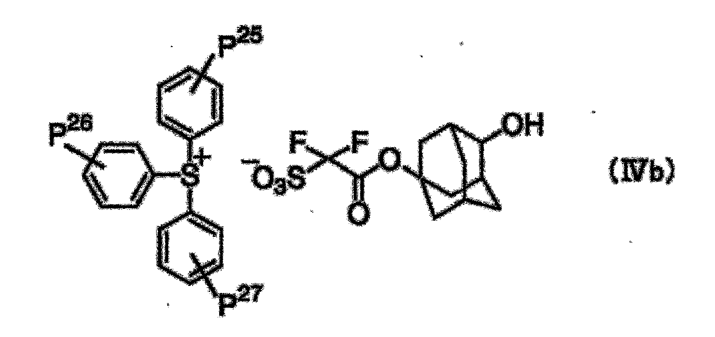

## Patentansprüche

1. Salz der Formel (L): 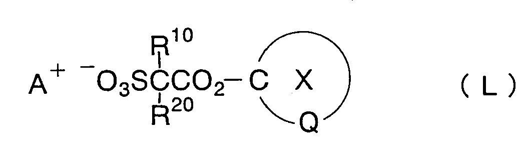 wobei Q einen Rest -CO- oder -C(OH)- darstellt, der Ring X einen monocyclischen oder polycyclischen Kohlenwasserstoffrest mit 3 bis 30 Kohlenstoffatomen darstellt, in welchem ein Wasserstoffatom an der Q-Position durch eine Hydroxylgruppe ersetzt ist, wenn Q für einen Rest -C(OH)- steht, oder in welchem zwei Wasserstoffatome an der Q-Position durch einen Rest =O ersetzt sind, wenn Q für einen Rest -CO- steht, und
wobei mindestens ein Wasserstoffatom in dem monocyclischen oder polycyclischen Kohlenwasserstoffrest gegebenenfalls durch einen 1 bis 6 Kohlenstoffatome aufweisenden Alkylrest, einen 1 bis 6 Kohlenstoffatome aufweisenden Alkoxyrest, einen 1 bis 4 Kohlenstoffatome aufweisenden Perfluoralkylrest, einen 1 bis 6 Kohlenstoffatome aufweisenden Hydroxyalkylrest, eine Hydroxylgruppe oder eine Cyanogruppe ersetzt sein kann; R¹⁰ und R²⁰ jeweils unabhängig voneinander ein Fluoratom oder einen Perfluoralkylrest mit 1 bis 6 Kohlenstoffatomen darstellen; und A⁺ ein organisches Gegenion darstellt.

2. Salz der Formel (LI): 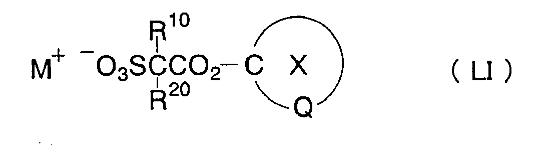 wobei Q einen Rest -CO- oder -C(OH)- darstellt; X einen monocyclischen oder polycyclischen Kohlenwasserstoffrest mit 3 bis 30 Kohlenstoffatomen darstellt, in welchem ein Wasserstoffatom an der Q-Position durch eine Hydroxylgruppe ersetzt ist, wenn Q für einen Rest -C(OH)- steht, oder in welchem zwei Wasserstoffatome an der Q-Position durch einen Rest =O ersetzt sind, wenn Q für einen Rest -CO- steht, und
wobei mindestens ein Wasserstoffatom in dem monocyclischen oder polycyclischen Kohlenwasserstoffrest gegebenenfalls durch einen 1 bis 6 Kohlenstoffatome aufweisenden Alkylrest, einen 1 bis 6 Kohlenstoffatome aufweisenden Alkoxyrest, einen 1 bis 4 Kohlenstoffatome aufweisenden Perfluoralkylrest, einen 1 bis 6 Kohlenstoffatome aufweisenden Hydroxyalkylrest, eine Hydroxylgruppe oder eine Cyanogruppe ersetzt sein kann; R¹⁰ und R²⁰ jeweils unabhängig voneinander ein Fluoratom oder einen Perfluoralkylrest mit 1 bis 6 Kohlenstoffatomen darstellen; und M für Li, Na, K oder Ag steht.

3. Salz gemäß Anspruch 2, wobei das Salz die Formel (V) aufweist: 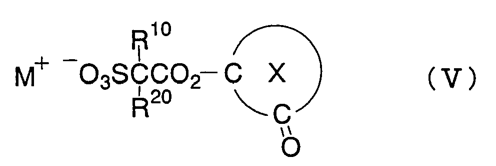 wobei X, R¹⁰, R²⁰ und M die in Anspruch 2 definierten Bedeutungen haben.

4. Salz gemäß Anspruch 2, wobei das Salz die Formel (VI) aufweist: 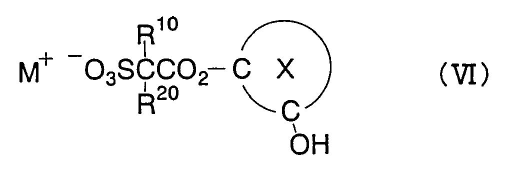 wobei X, R¹⁰, R²⁰ und M die in Anspruch 2 definierten Bedeutungen haben.

5. Verfahren zur Herstellung eines Salzes der Formel (LI) 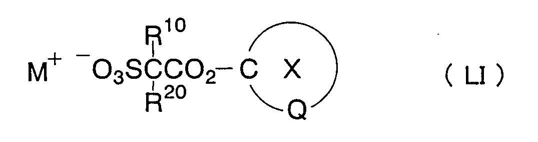 wobei Q einen Rest -CO- oder -C(OH)- darstellt; X einen monocyclischen oder polycyclischen Kohlenwasserstoffrest mit 3 bis 30 Kohlenstoffatomen darstellt, in welchem ein Wasserstoffatom an der Q-Position durch eine Hydroxylgruppe ersetzt ist, wenn Q für einen Rest -C(OH)- steht, oder in welchem zwei Wasserstoffatome an der Q-Position durch einen Rest =O ersetzt sind, wenn Q für einen Rest -CO- steht, und
wobei mindestens ein Wasserstoffatom in dem monocyclischen oder polycyclischen Kohlenwasserstoffrest gegebenenfalls durch einen 1 bis 6 Kohlenstoffatome aufweisenden Alkylrest, einen 1 bis 6 Kohlenstoffatome aufweisenden Alkoxyrest, einen 1 bis 4 Kohlenstoffatome aufweisenden Perfluoralkylrest, einen 1 bis 6 Kohlenstoffatome aufweisenden Hydroxyalkylrest, eine Hydroxylgruppe oder eine Cyanogruppe ersetzt sein kann; R¹⁰ und R²⁰ jeweils unabhängig voneinander ein Fluoratom oder einen Perfluoralkylrest mit 1 bis 6 Kohlenstoffatomen darstellen; und M für Li, Na, K oder Ag steht,
wobei das Verfahren die Veresterung eines Alkohols der Formel (LII) 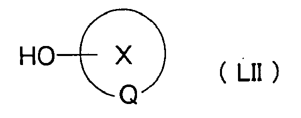 wobei X und Q die vorstehend definierten Bedeutungen haben,
mit einer Carbonsäure der Formel (IX) 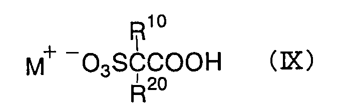 wobei R¹⁰, R²⁰ und M die vorstehend definierten Bedeutungen haben,
umfasst.

6. Verfahren zur Herstellung eines Salzes der Formel (II) 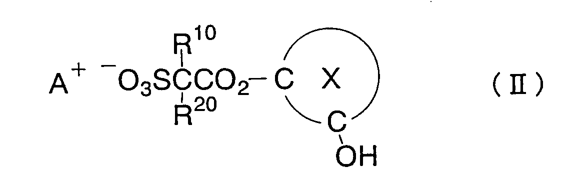 wobei der Ring X einen monocyclischen oder polycyclischen Kohlenwasserstoffrest mit 3 bis 30 Kohlenstoffatomen darstellt, in welchem ein Wasserstoffatom an der Q-Position durch eine Hydroxylgruppe ersetzt ist, wenn Q für einen Rest -C(OH)- steht, oder in welchem zwei Wasserstoffatome an der Q-Position durch einen Rest =O ersetzt sind, wenn Q für einen Rest -CO- steht, und wobei mindestens ein Wasserstoffatom in dem monocyclischen oder polycyclischen Kohlenwasserstoffrest gegebenenfalls durch einen 1 bis 6 Kohlenstoffatome aufweisenden Alkylrest, einen 1 bis 6 Kohlenstoffatome aufweisenden Alkoxyrest, einen 1 bis 4 Kohlenstoffatome aufweisenden Perfluoralkylrest, einen 1 bis 6 Kohlenstoffatome aufweisenden Hydroxyalkylrest, eine Hydroxylgruppe oder eine Cyanogruppe ersetzt sein kann; R¹⁰ und R²⁰ jeweils unabhängig voneinander ein Fluoratom oder einen Perfluoralkylrest mit 1 bis 6 Kohlenstoffatomen darstellen; und A⁺ ein organisches Gegenion darstellt,
wobei das Verfahren die Reduzierung eines Salzes der Formel (I) 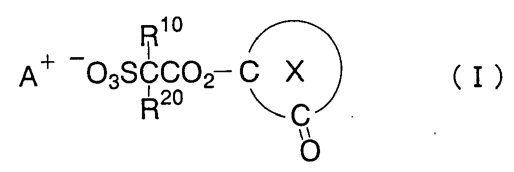 umfasst,
wobei X, A, R¹⁰ und R²⁰ die vorstehend definierten Bedeutungen haben.

7. Verfahren zur Herstellung eines Salzes der Formel (VI) 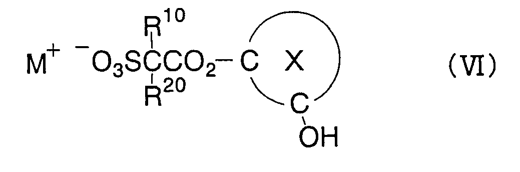 wobei X einen monocyclischen oder polycyclischen Kohlenwasserstoffrest mit 3 bis 30 Kohlenstoffatomen darstellt, in welchem ein Wasserstoffatom an der Q-Position durch eine Hydroxylgruppe ersetzt ist, wenn Q für einen Rest -C(OH)- steht, oder in welchem zwei Wasserstoffatome an der Q-Position durch einen Rest =O ersetzt sind, wenn Q für einen Rest -CO- steht, und wobei mindestens ein Wasserstoffatom in dem monocyclischen oder polycyclischen Kohlenwasserstoffrest gegebenenfalls durch einen 1 bis 6 Kohlenstoffatome aufweisenden Alkylrest, einen 1 bis 6 Kohlenstoffatome aufweisenden Alkoxyrest, einen 1 bis 4 Kohlenstoffatome aufweisenden Perfluoralkylrest, einen 1 bis 6 Kohlenstoffatome aufweisenden Hydroxyalkylrest, eine Hydroxylgruppe oder eine Cyanogruppe ersetzt sein kann; R¹⁰ und R²⁰ jeweils unabhängig voneinander ein Fluoratom oder einen Perfluoralkylrest mit 1 bis 6 Kohlenstoffatomen darstellen; und M für Li, Na, K oder Ag steht,
wobei das Verfahren die Reduzierung eines Salzes der Formel (V) 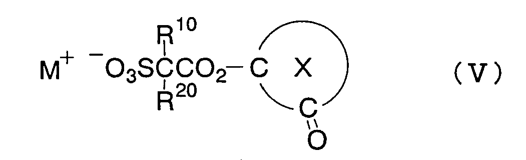 umfasst,
wobei X, R¹⁰, R²⁰ und M die vorstehend definierten Bedeutungen haben.

8. Verfahren zur Herstellung eines Salzes der Formel (LI) 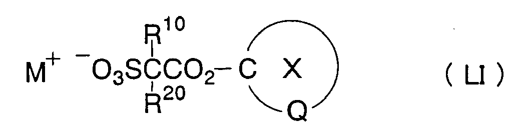 wobei Q einen Rest -CO- oder -C(OH)- darstellt; X einen monocyclischen oder polycyclischen Kohlenwasserstoffrest mit 3 bis 30 Kohlenstoffatomen darstellt, in welchem ein Wasserstoffatom an der Q-Position durch eine Hydroxylgruppe ersetzt ist, wenn Q für einen Rest -C(OH)- steht, oder in welchem zwei Wasserstoffatome an der Q-Position durch einen Rest =O ersetzt sind, wenn Q für einen Rest -CO- steht, und
wobei mindestens ein Wasserstoffatom in dem monocyclischen oder polycyclischen Kohlenwasserstoffrest gegebenenfalls durch einen 1 bis 6 Kohlenstoffatome aufweisenden Alkylrest, einen 1 bis 6 Kohlenstoffatome aufweisenden Alkoxyrest, einen 1 bis 4 Kohlenstoffatome aufweisenden Perfluoralkylrest, einen 1 bis 6 Kohlenstoffatome aufweisenden Hydroxyalkylrest, eine Hydroxylgruppe oder eine Cyanogruppe ersetzt sein kann; R¹⁰ und R²⁰ jeweils unabhängig voneinander ein Fluoratom oder einen Perfluoralkylrest mit 1 bis 6 Kohlenstoffatomen darstellen; und M für Li, Na, K oder Ag steht,
wobei das Verfahren die Veresterung eines Alkohols der Formel (LII) 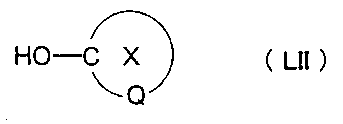 wobei X und Q die vorstehend definierten Bedeutungen haben,
mit einer Carbonsäure der Formel (X) 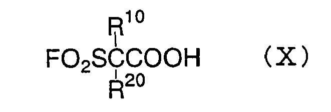 wobei R¹⁰ und R²⁰ die vorstehend definierten Bedeutungen haben,
und Hydrolysieren mit MOH, wobei M für Li, Na, K oder Ag steht, umfasst.

9. Verfahren zur Herstellung eines Salzes der Formel (L) 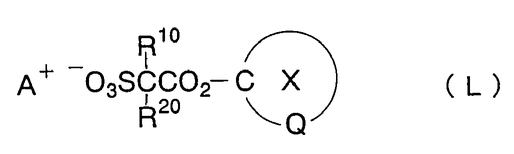 wobei Q einen Rest -CO- oder -C(OH)- darstellt; der Ring X einen monocyclischen oder polycyclischen Kohlenwasserstoffrest mit 3 bis 30 Kohlenstoffatomen darstellt, in welchem ein Wasserstoffatom an der Q-Position durch eine Hydroxylgruppe ersetzt ist, wenn Q für einen Rest -C(OH)- steht, oder in welchem zwei Wasserstoffatome an der Q-Position durch einen Rest =O ersetzt sind, wenn Q für einen Rest -CO- steht, und
wobei mindestens ein Wasserstoffatom in dem monocyclischen oder polycyclischen Kohlenwasserstoffrest gegebenenfalls durch einen 1 bis 6 Kohlenstoffatome aufweisenden Alkylrest, einen 1 bis 6 Kohlenstoffatome aufweisenden Alkoxyrest, einen 1 bis 4 Kohlenstoffatome aufweisenden Perfluoralkylrest, einen 1 bis 6 Kohlenstoffatome aufweisenden Hydroxyalkylrest, eine Hydroxylgruppe oder eine Cyanogruppe ersetzt sein kann; R¹⁰ und R²⁰ jeweils unabhängig voneinander ein Fluoratom oder einen Perfluoralkylrest mit 1 bis 6 Kohlenstoffatomen darstellen; und A⁺ ein organisches Gegenion darstellt,
wobei das Verfahren die Umsetzung eines Salzes der Formel (LI) 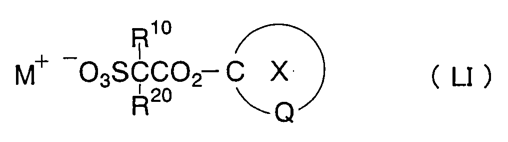 wobei M für Li, Na, K oder Ag steht und Q, R¹⁰, R²⁰ und X die vorstehend definierten Bedeutungen haben,
mit einer Verbindung der Formel (XI)
A⁺Z⁻ (XI)
wobei Z für F, Cl, Br, I, BF₄, AsF₆, SbF₆, PF₆ oder ClO₄ steht und A⁺ die vorstehend beschriebene Bedeutung hat,
umfasst.

10. Chemisch verstärkte Resistzusammensetzung, umfassend
ein Salz der Formel (L) 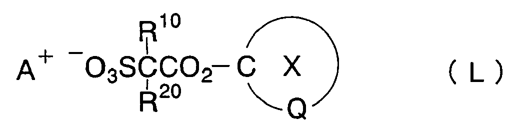 wobei Q einen Rest -CO- oder -C(OH)- darstellt; der Ring X einen monocyclischen oder polycyclischen Kohlenwasserstoffrest mit 3 bis 30 Kohlenstoffatomen darstellt, in welchem ein Wasserstoffatom an der Q-Position durch eine Hydroxylgruppe ersetzt ist, wenn Q für einen Rest -C(OH)- steht, oder in welchem zwei Wasserstoffatome an der Q-Position durch einen Rest =O ersetzt sind, wenn Q für einen Rest -CO- steht, und
wobei mindestens ein Wasserstoffatom in dem monocyclischen oder polycyclischen Kohlenwasserstoffrest gegebenenfalls durch einen 1 bis 6 Kohlenstoffatome aufweisenden Alkylrest, einen 1 bis 6 Kohlenstoffatome aufweisenden Alkoxyrest, einen 1 bis 4 Kohlenstoffatome aufweisenden Perfluoralkylrest, einen 1 bis 6 Kohlenstoffatome aufweisenden Hydroxyalkylrest, eine Hydroxylgruppe oder eine Cyanogruppe ersetzt sein kann; R¹⁰ und R²⁰ jeweils unabhängig voneinander ein Fluoratom oder einen Perfluoralkylrest mit 1 bis 6 Kohlenstoffatomen darstellen; und A⁺ ein organisches Gegenion darstellt, und
ein Harz, welches eine Struktureinheit mit einem säureempimdlichen Rest enthält und wobei das Harz in einer alkalischen, wässrigen Lösung unlöslich oder schwer löslich ist, aber durch die Wirkung einer Säure in einer alkalischen, wässrigen Lösung löslich wird.

11. Zusammensetzung gemäß Anspruch 10, wobei das Harz eine Struktureinheit enthält, welche abgeleitet ist aus einem Monomer mit einem großen und säureempfindlichen Rest.

12. Zusammensetzung gemäß Anspruch 11, wobei der große und säureempfindliche Rest ein 2-Alkyl-2-adamantyl-Rest oder ein 1-(1-Adamantyl)-1-alkylalkyl-Rest ist.

13. Zusammensetzung gemäß Anspruch 11, wobei das Monomer mit dem großen und säureempfindlichen Rest 2-Alkyl-2-adamantyl(meth)acrylat, 1-(1-Adamantyl)-1-alkylalkyl(meth)acrylat, 2-Alkyl-2-adamantyl-5-norbornen-2-carboxylat, 1-(1-Adamantyl)-1-alkylalkyl-5-norbornen-2-carboxylat, 2-Alkyl-2-adamantyl-α-chloracrylat oder 1-(1-Adamantyl)-1-alkylalkyl-α-chloracrylat ist.

14. Zusammensetzung gemäß einem der Ansprüche 10 bis 13, wobei die Zusammensetzung weiterhin eine basische Verbindung umfasst.

15. Salz, Verfahren oder Zusammensetzung gemäß einem der Ansprüche 1 bis 14, wobei jeder der Reste R¹⁰ und R²⁰ ein Fluoratom oder eine Trifluormethylgruppe ist.

16. Salz, Verfahren oder Zusammensetzung gemäß einem der Ansprüche 1, 10, 11, 12, 13, 14 und 15, wobei das Salz die folgende Formel (I) aufweist 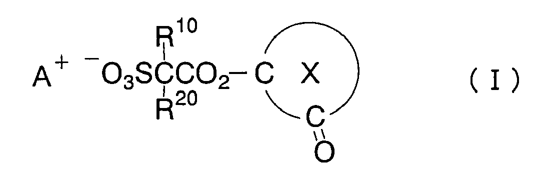 wobei X, A, R¹⁰ und R²⁰ die in Anspruch 10 definierten Bedeutungen haben.

17. Salz, Verfahren oder Zusammensetzung gemäß einem der Ansprüche 1, 10, 11, 12, 13, 14 und 15, wobei das Salz die folgende Formel (II) aufweist 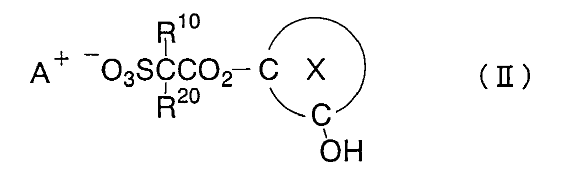 wobei X, A, R¹⁰ und R²⁰ die in Anspruch 10 definierten Bedeutungen haben.

18. Salz, Verfahren oder Zusammensetzung gemäß einem der Ansprüche 1, 10, 11, 12, 13, 14, 15, 16 und 17, wobei A⁺ mindestens ein Kation ausgewählt aus der Formel (IIIa), der Formel (IIIb), der Formel (IIIc) und der Formel (IIId) ist:
ein Kation der Formel (IIIa) 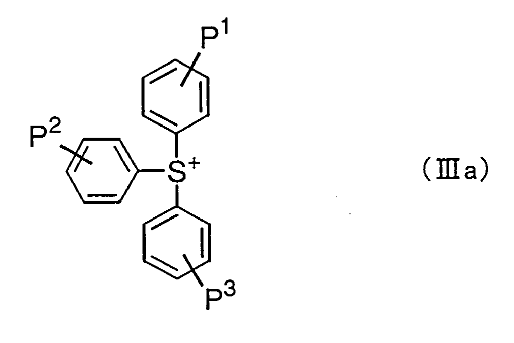 wobei P¹, P² und P³ jeweils unabhängig voneinander ein Wasserstoffatom, eine Hydroxylgruppe, einen Alkylrest mit 1 bis 12 Kohlenstoffatomen oder einen Alkoxyrest mit 1 bis 12 Kohlenstoffatomen darstellen;
ein Kation der Formel (IIIb) 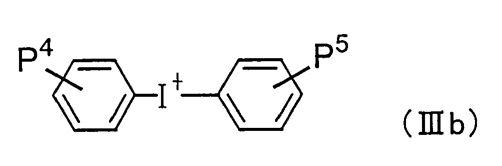 wobei P⁴ und P⁵ jeweils unabhängig voneinander ein Wasserstoffatom, eine Hydroxylgruppe, einen Alkylrest mit 1 bis 12 Kohlenstoffatomen oder einen Alkoxyrest mit 1 bis 12 Kohlenstoffatomen darstellen;
ein Kation der Formel (IIIc) 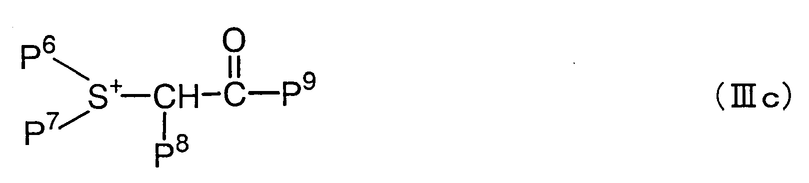 wobei P⁶ und P⁷ jeweils unabhängig voneinander einen Alkylrest mit 1 bis 12 Kohlenstoffatomen oder einen Cycloalkylrest mit 3 bis 12 Kohlenstoffatomen darstellen, oder P⁶ und P⁷ verbunden sind, um einen zweiwertigen acyclischen Kohlenwasserstoffrest mit 3 bis 12 Kohlenstoffatomen zu bilden, welcher zusammen mit dem benachbarten S⁺ einen Ring bildet, und wobei mindestens ein Rest -CH₂- in dem zweiwertigen acyclischen Kohlenwasserstoffrest gegebenenfalls durch -CO-, -O- oder -S- ersetzt ist, P⁸ ein Wasserstoffatom darstellt, P⁹ einen Alkylrest mit 1 bis 12 Kohlenstoffatomen, einen Cycloalkylrest mit 3 bis 12 Kohlenstoffatomen oder einen gegebenenfalls substituierten aromatischen Ring darstellt, oder P⁸ und P⁹ verbunden sind, um einen zweiwertigen acyclischen Kohlenwasserstoffrest zu bilden, welcher zusammen mit dem benachbarten Rest -CHCO- 2-Oxocycloalkyl bildet, und mindestens ein Rest -CH₂- in dem zweiwertigen acyclischen Kohlenwasserstoffrest gegebenenfalls durch -CO-, -O- oder -S- ersetzt ist;
ein Kation der Formel (IIId) 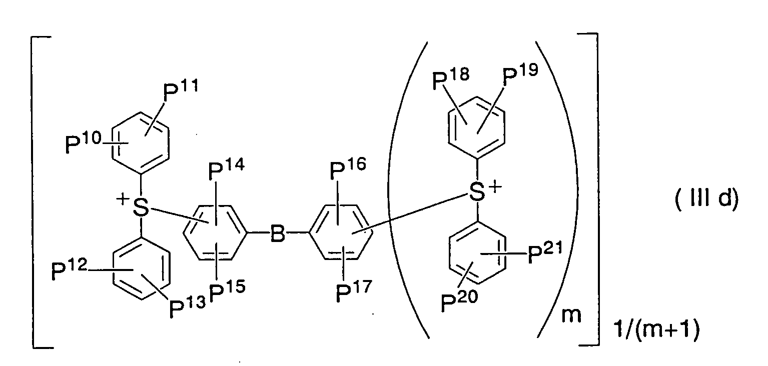 wobei P¹⁰, P¹¹, P¹², P¹³, P¹⁴, P¹⁵, P¹⁶, P¹⁷, P¹⁸, P¹⁹, P²⁰ und P²¹ jeweils unabhängig voneinander ein Wasserstoffatom, eine Hydroxylgruppe, einen Alkylrest mit 1 bis 12 Kohlenstoffatomen oder einen Alkoxyrest mit 1 bis 12 Kohlenstoffatomen darstellen, B ein Schwefelatom oder Sauerstoffatom darstellt und m für 0 oder 1 steht.

19. Salz, Verfahren oder Zusammensetzung gemäß Anspruch 18, wobei A⁺ ein Gegenion der Formel (IIIe) ist: 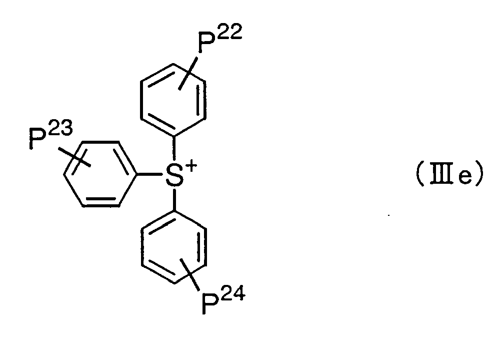 wobei P²², P²³ und P²⁴ jeweils unabhängig voneinander ein Wasserstoffatom oder einen Alkylrest mit 1 bis 4 Atomen darstellen.

20. Salz, Verfahren oder Zusammensetzung gemäß einem der Ansprüche 1, 10, 11, 12, 13, 14, 15, 16, 17, 18 und 19, wobei der Ring X ein Cycloalkylrest mit 4 bis 8 Kohlenstoffatomen, ein Adamantylrest oder ein Norbornylrest ist, mit der Maßgabe, dass in jedem der Reste ein Wasserstoffatom an der Q-Position durch einen Rest -OH ersetzt ist, wenn Q für einen Rest -C(OH)- steht und zwei Wasserstoffatome an der Q-Position durch =O ersetzt sind, wenn Q für einen Rest -CO- steht, und mit der Maßgabe, dass mindestens ein Wasserstoffatom in jedem der Reste gegebenenfalls durch einen 1 bis 6 Kohlenstoffatome aufweisenden Alkylrest, einen 1 bis 6 Kohlenstoffatome aufweisenden Alkoxyrest, einen 1 bis 4 Kohlenstoffatome aufweisenden Perfluoralkylrest, einen 1 bis 6 Kohlenstoffatome aufweisenden Hydroxyalkylrest, eine Hydroxylgruppe oder eine Cyanogruppe ersetzt sein kann.

21. Salz oder Zusammensetzung gemäß einem der Ansprüche 1, 10, 11, 12, 13 und 14, wobei das Salz das der Formel (IVa) ist: 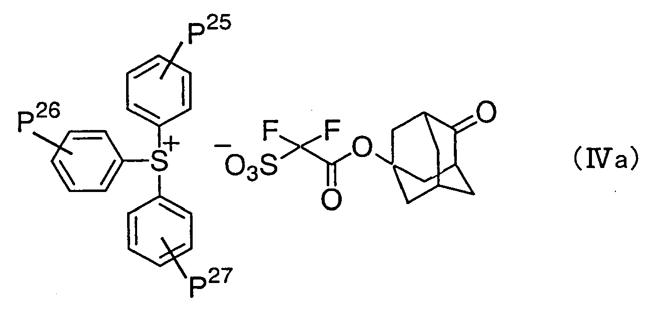 wobei P²⁵, P²⁶ und P²⁷ jeweils unabhängig voneinander Wasserstoffatome oder einen Alkylrest mit 1 bis 4 Kohlenstoffatomen darstellen.

22. Salz oder Zusammensetzung gemäß einem der Ansprüche 1, 10, 11, 12, 13 und 14, wobei das Salz das der Formel (IVb) ist: 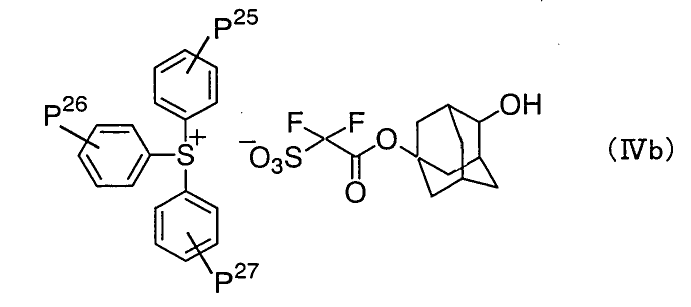 wobei P²⁵, P²⁶ und P²⁷ die in Anspruch 21 definierten Bedeutungen haben.

## Revendications

1. Sel de la formule (L) 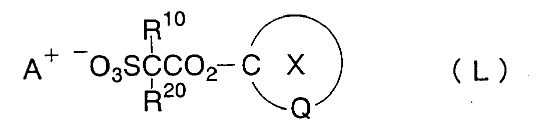 dans laquelle Q représente un groupe -CO- ou un groupe -C(OH)-, le noyau X représente un groupe hydrocarboné monocyclique ou polycyclique comportant de 3 à 30 atomes de carbone, dans lequel un atome d'hydrogène est remplacé par un groupe hydroxyle en position Q lorsque Q est un groupe -C(OH)- ou dans lequel deux atomes d'hydrogène sont remplacés par un groupe =O en position Q lorsque Q est un groupe -CO-, et au moins un atome d'hydrogène dans le groupe hydrocarboné monocyclique ou polycyclique peut facultativement être remplacé par un groupe alkyle comportant de 1 à 6 atomes de carbone, un groupe alcoxy comportant de 1 à 6 atomes de carbone, un groupe perfluoroalkyle comportant de 1 à 4 atomes de carbone, un groupe hydroxyalkyle comportant de 1 à 6 atomes de carbone, un groupe hydroxyle ou un groupe cyano, R¹⁰ et R²⁰ représentent chacun indépendamment l'un de l'autre un atome de fluor ou un groupe perfluoroalkyle comportant de 1 à 6 atomes de carbone, et A⁺ représente un contre-ion organique.

2. Sel de la formule (LI) 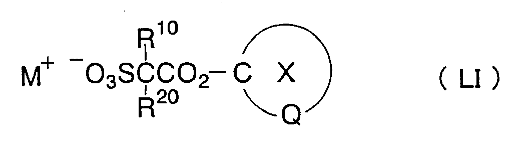 dans laquelle Q représente un groupe -CO- ou un groupe -C(OH)-, X représente un groupe hydrocarboné monocyclique ou polycyclique comportant de 3 à 30 atomes de carbone, dans lequel un atome d'hydrogène est remplacé par un groupe hydroxyle en position Q lorsque Q est un groupe -C(OH)- ou dans lequel deux atomes d'hydrogène sont remplacés par un groupe =O en position Q lorsque Q est un groupe -CO-, et au moins un atome d'hydrogène dans le groupe hydrocarboné monocyclique ou polycyclique peut facultativement être remplacé par un groupe alkyle comportant de 1 à 6 atomes de carbone, un groupe alcoxy comportant de 1 à 6 atomes de carbone, un groupe perfluoroalkyle comportant de 1 à 4 atomes de carbone, un groupe hydroxyalkyle comportant de 1 à 6 atomes de carbone, un groupe hydroxyle ou un groupe cyano, R¹⁰ et R²⁰ représentent chacun indépendamment l'un de l'autre un atome de fluor ou un groupe perfluoroalkyle comportant de 1 à 6 atomes de carbone, et M représente Li, Na, K ou Ag.

3. Sel suivant la revendication 2, dans lequel le sel présente la formule ( V) 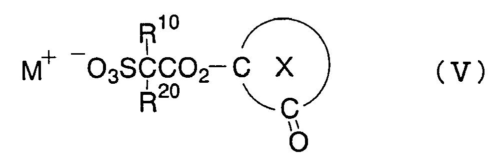 dans laquelle X, R¹⁰, R²⁰ et M ont les mêmes significations que celles définies dans la revendication 2.

4. Sel suivant la revendication 2, dans lequel le sel présente la formule (VI) 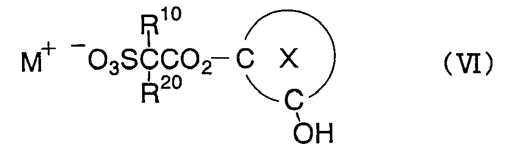 dans laquelle X, R¹⁰, R²⁰ et M ont les mêmes significations que celles définies dans la revendication 2.

5. Procédé de production d'un sel de la formule (LI) 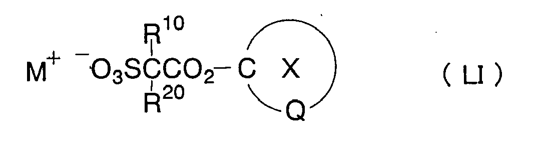 dans laquelle Q représente un groupe -CO- ou un groupe -C(OH)-, X représente un groupe hydrocarboné monocyclique ou polycyclique comportant de 3 à 30 atomes de carbone, dans lequel un atome d'hydrogène est remplacé par un groupe hydroxyle en position Q lorsque Q est un groupe -C(OH)- ou dans lequel deux atomes d'hydrogène sont remplacés par un groupe =O en position Q lorsque Q est un groupe -CO- et au moins un atome d'hydrogène dans le groupe hydrocarboné monocyclique ou polycyclique peut facultativement être remplacé par un groupe alkyle comportant de 1 à 6 atomes de carbone, un groupe alcoxy comportant de 1 à 6 atomes de carbone, un groupe perfluoroalkyle comportant de 1 à 4 atomes de carbone, un groupe hydroxyalkyle comportant de 1 à 6 atomes de carbone, un groupe hydroxyle ou un groupe cyano, R¹⁰ et R²⁰ représentent chacun indépendamment l'un de l'autre un atome de fluor ou un groupe perfluoroalkyle comportant de 1 à 6 atomes de carbone, et M représente Li, Na, K ou Ag et qui comprend l'estérification d'un alcool de la formule (LII) 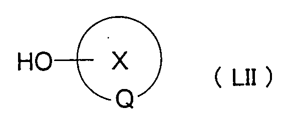 dans laquelle X et Q ont les mêmes significations que celles définies plus haut,
avec un acide carboxylique de la formule (IX) 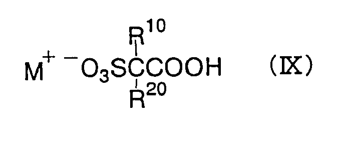 dans laquelle R¹⁰, R²⁰ et M ont les mêmes significations que celles définies plus haut.

6. Procédé de production d'un sel de la formule (II) 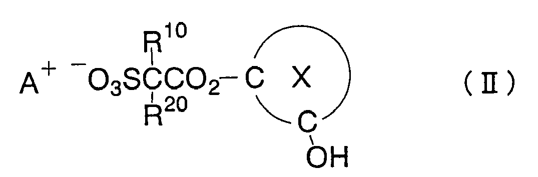 dans laquelle X représente un groupe hydrocarboné monocyclique ou polycyclique comportant de 3 à 30 atomes de carbone, où un atome d'hydrogène est remplacé par un groupe hydroxyle en position Q lorsque Q est un groupe -C(OH)- ou dans lequel deux atomes d'hydrogène sont remplacés par un groupe =O en position Q lorsque Q est un groupe -CO-, et au moins un atome d'hydrogène dans le groupe hydrocarboné monocyclique ou polycyclique peut facultativement être remplacé par un groupe alkyle comportant de 1 à 6 atomes de carbone, un groupe alcoxy comportant de 1 à 6 atomes de carbone, un groupe perfluoroalkyle comportant de 1 à 4 atomes de carbone, un groupe hydroxyalkyle comportant de 1 à 6 atomes de carbone, un groupe hydroxyle ou un groupe cyano, R¹⁰ et R²⁰ représentent chacun indépendamment l'un de l'autre un atome de fluor ou un groupe perfluoroalkyle comportant de 1 à 6 atomes de carbone, et A⁺ représente un contre-ion organique,
comprenant la réduction d'un sel de la formule (I) 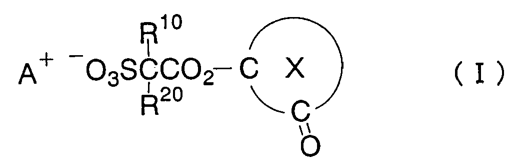 dans laquelle X, A, R¹⁰ et R²⁰ ont les mêmes significations que celle définies plus haut.

7. Procédé de production d'un sel de la formule (VI) 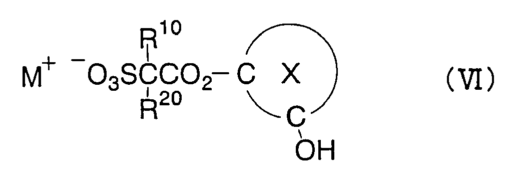 dans laquelle X représente un groupe hydrocarboné monocyclique ou polycyclique comportant de 3 à 30 atomes de carbone, dans lequel un atome d'hydrogène est remplacé par un groupe hydroxyle en position Q lorsque Q est un groupe -C(OH)- ou dans lequel deux atomes d'hydrogène sont remplacés par un groupe =O en position Q lorsque Q est un groupe -CO-, et au moins un atome d'hydrogène du groupe hydrocarboné monocyclique ou polycyclique peut facultativement être remplacé par un groupe alkyle comportant de 1 à 6 atomes de carbone, un groupe alcoxy comportant de 1 à 6 atomes de carbone, un groupe perfluoroalkyle comportant de 1 à 4 atomes de carbone, un groupe hydroxyalkyle comportant de 1 à 6 atomes de carbone, un groupe hydroxyle ou un groupe cyano, R¹⁰ et R²⁰ représentent chacun indépendamment l'un de l'autre un atome de fluor ou un groupe perfluoroalkyle comportant de 1 à 6 atomes de carbone, et M représente Li, Na, K ou Ag,
comprenant la réduction d'un sel de la formule (V) 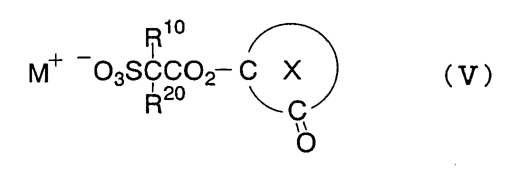 dans laquelle X, R¹⁰, R²⁰ et M ont les mêmes significations que celles définies plus haut.

8. Procédé de production d'un sel de la formule (LI) 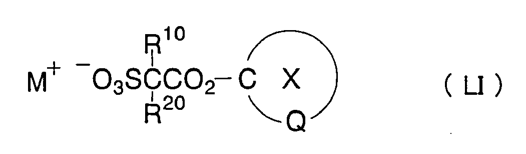 dans laquelle Q représente un groupe -CO- ou un groupe -C(OH)-, X représente un groupe hydrocarboné monocyclique ou polycyclique comportant de 3 à 30 atomes de carbone dans lequel un atome d'hydrogène est remplacé par un groupe hydroxyle en position Q lorsque Q est un groupe -C(OH)- ou dans lequel deux atomes d'hydrogène sont remplacés par un groupe =O en position Q lorsque Q est un groupe -CO-, et au moins un atome d'hydrogène du groupe hydrocarboné monocyclique ou polycyclique peut facultativement être remplacé par un groupe alkyle comportant de 1 à 6 atomes de carbone, un groupe alcoxy comportant de 1 à 6 atomes de carbone, un groupe perfluoroalkyle comportant de 1 à 4 atomes de carbone, un groupe hydroxyalkyle comportant de 1 à 6 atomes de carbone, un groupe hydroxyle ou un groupe cyano, R¹⁰ et R²⁰ représentent chacun indépendamment l'un de l'autre un atome de fluor ou un groupe perfluoroalkyle comportant de 1 à 6 atomes de carbone, et M représente Li, Na, K ou Ag,
comprenant l'estérification d'un alcool de la formule (LII) 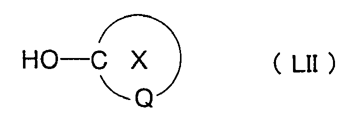 dans laquelle X et Q ont la même signification que définie ci-avant,
avec un acide carboxylique de la formule (X) 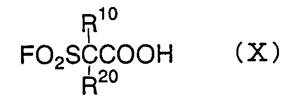 dans laquelle R¹⁰ et R²⁰ ont les mêmes significations que celles définies plus haut,
et une hydrolyse avec du MOH, où M représente Li, Na, K ou Ag.

9. Procédé de production d'un sel de la formule (L) 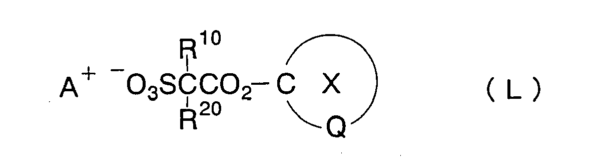 dans laquelle Q représente un groupe -CO- ou un groupe -C(OH)-, le noyau X représente un groupe hydrocarboné monocyclique ou polycyclique comportant de 3 à 30 atomes de carbone, dans lequel un atome d'hydrogène est remplacé par un groupe hydroxyle en position Q lorsque Q est un groupe -C(OH)- ou dans lequel deux atomes d'hydrogène sont remplacés par un groupe =O en position Q lorsque Q est un groupe -CO-, et au moins un atome d'hydrogène dans le groupe hydrocarboné monocyclique ou polycyclique peut facultativement être remplacé par un groupe alkyle comportant de 1 à 6 atomes de carbone, un groupe alcoxy comportant de 1 à 6 atomes de carbone, un groupe perfluoroalkyle comportant de 1 à 4 atomes de carbone, un groupe hydroxyalkyle comportant de 1 à 6 atomes de carbone, un groupe hydroxyle ou un groupe cyano, R¹⁰ et R²⁰ représentent chacun indépendamment l'un de l'autre un atome de fluor ou un groupe perfluoroalkyle comportant de 1 à 6 atomes de carbone, et A⁺ représente un contre-ion organique,
comprenant la réaction d'un sel de la formule (LI) 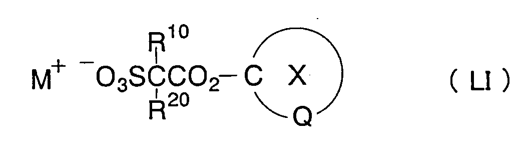 dans laquelle M représente Li, Na, K ou Ag, et Q, R¹⁰, R²⁰ et X ont les mêmes significations que celles définies plus haut,
avec un composé de la formule (XI)
A⁺Z⁻ (XI)
dans laquelle Z représente F, Cl, Br, l, BF₄, AsF₆, SbF₆, PF₆, ou ClO₄, et A⁺ a la même signification que celle définie plus haut.

10. Composition de résiste chimiquement amplifiée comprenant un sel de la formule (L) 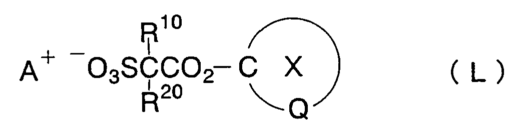 dans laquelle Q représente un groupe -CO- ou un groupe -C(OH)-, le noyau X représente un groupe hydrocarboné monocyclique ou polycyclique comportant de 3 à 30 atomes de carbone, dans lequel un atome d'hydrogène est remplacé par un groupe hydroxyle en position Q lorsque Q est un groupe -C(OH)- ou dans lequel deux atomes d'hydrogène sont remplacés par un groupe =O en position Q lorsque Q est un groupe -CO- et au moins un atome d'hydrogène dans le groupe hydrocarboné monocyclique ou polycyclique peut facultativement être remplacé par un groupe alkyle comportant de 1 à 6 atomes de carbone, un groupe alcoxy comportant de 1 à 6 atomes de carbone, un groupe perfluoroalkyle comportant de 1 à 4 atomes de carbone, un groupe hydroxyalkyle comportant de 1 à 6 atomes de carbone, un groupe hydroxyle ou un groupe cyano, R¹⁰ et R²⁰ représentent chacun indépendamment l'un de l'autre un atome de fluor ou un groupe perfluoroalkyle comportant de 1 à 6 atomes de carbone, et A⁺ représente un contre-ion organique, et
une résine qui contient une unité structurelle possédant un groupe acidolabile, laquelle résine est insoluble ou peu soluble dans une solution aqueuse alcaline, mais devient soluble dans une solution aqueuse alcaline sous action d'un acide.

11. Composition suivant la revendication 10, dans laquelle la résine contient une unité structurelle dérivée d'un monomère possédant un groupe acidolabile et volumineux.

12. Composition suivant la revendication 11, dans laquelle le groupe acidolabile et volumineux est un groupe 2-alkyle-2-adamantyle ou un groupe 1-(1-adamantyl)-1-alkylalkyle.

13. Composition suivant la revendication 11, dans laquelle le monomère possédant un groupe acidolabile et volumineux est du (méth)acrylate de 2-alkyl-2-adamantyle, du (méth)acrylate de 1-(1-adamantyl)-1-alkylalkyle, du 2-carboxylate de 2-alkyl-2-adamantyl-5-norbornène, du 2-carboxylate de 1-(1-adamantyl)-1-alkylalkyl-5-norbornène, de l'α-chloroacrylate de 2-alkyl-2-adamantyle ou de l'α-chloroacrylate de 1-(1-adamantyl)-1-alkylalkyle.

14. Composition suivant l'une quelconque des revendications 10 à 13, dans laquelle la composition comprend en outre un composé basique.

15. Sel, procédé ou composition suivant l'une quelconque des revendications 1 à 14, où chacun de R¹⁰ et R²⁰ est un atome de fluor ou un groupe trifluorométhyle.

16. Sel, procédé ou composition suivant l'une quelconque des revendications 1, 10, 11, 12, 13, 14 et 15, où le sel présente la formule (I) ci-après 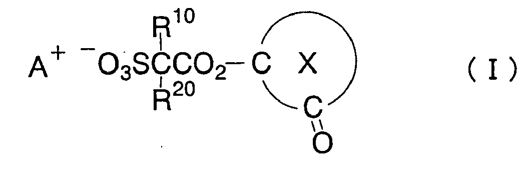 dans laquelle X, A, R¹⁰ et R²⁰ ont les mêmes significations que celles définies dans la revendication 10.

17. Sel, procédé ou composition suivant l'une quelconque des revendications 1, 10, 11, 12, 13, 14 et 15, où le sel présente la formule (II) ci-après 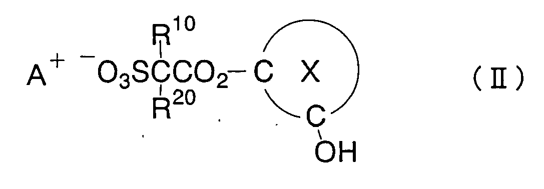 dans laquelle X, A, R¹⁰ et R²⁰ ont les mêmes significations que celles définies dans la revendication 10.

18. Sel, procédé ou composition suivant l'une quelconque des revendications 1, 10, 11, 12, 13, 14, 15, 16 et 17, où A⁺ est au moins un cation choisi dans le groupe consistant en la formule (IIIa), la formule (IIIb), la formule (IIIc) et la formule (IIId) :
un cation de la formule (IIIa) 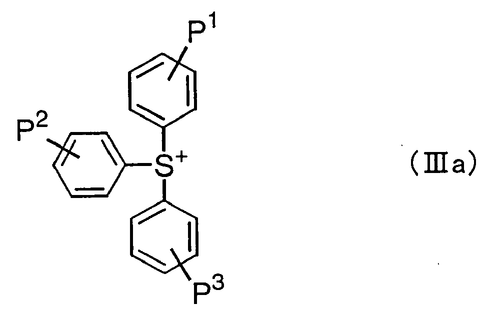 dans laquelle P¹, P² et P³ représentent chacun indépendamment les uns des autres un atome d'hydrogène, un groupe hydroxyle, un groupe alkyle comportant de 1 à 12 atomes de carbone ou un groupe alcoxy comportant de 1 à 12 atomes de carbone,
un cation de la formule (IIIb) 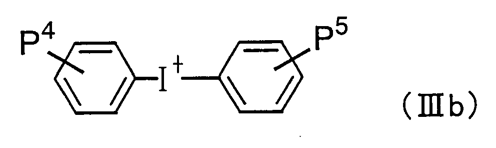 dans laquelle P⁴ et P⁵ représentent chacun indépendamment l'un de l'autre un atome d'hydrogène, un groupe hydroxyle, un groupe alkyle comportant de 1 à 12 atomes de carbone ou un groupe alcoxy comportant de 1 à 12 atomes de carbone,
un cation de la formule (IIIc) 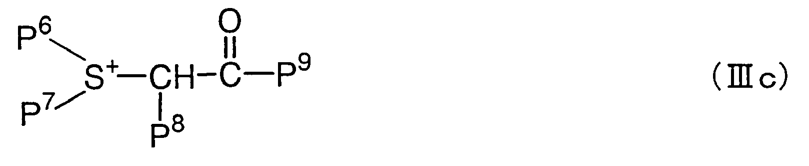 dans laquelle P⁶ et P⁷ représentent chacun indépendamment l'un de l'autre un alkyle comportant de 1 à 12 atomes de carbone ou cycloalkyle comportant de 3 à 12 atomes de carbone, ou bien P⁶ et P⁷ se lient pour former un groupe hydrocarboné acyclique divalent comportant de 3 à 12 atomes de carbone, qui forme un noyau avec le S⁺ adjacent, et au moins un -CH₂- du groupe hydrocarboné acyclique divalent est éventuellement remplacé par -CO-, -O- ou -S-, P⁸ représente de l'hydrogène, P⁹ représente un alkyle comportant de 1 à 12 atomes de carbone, un cycloalkyle comportant de 3 à 12 atomes de carbone ou un groupe cyclique aromatique éventuellement substitué, ou bien P⁸ et P⁹ se lient pour former un groupe hydrocarboné acyclique divalent qui forme un 2-oxocycloalkyle avec le -CHCO- adjacent, et au moins un -CH₂- du groupe hydrocarboné acyclique divalent est éventuellement remplacé par -CO-, -O- ou -S-,
un cation de la formule (IIId) 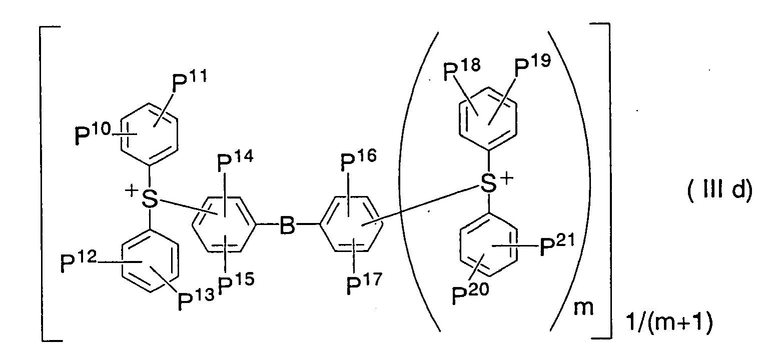 dans laquelle P¹⁰, P¹¹, P^{12,} P¹³, P^{14,} P¹⁵, P¹⁶, P¹⁷, P¹⁸, P¹⁹, P²⁰ et P²¹ représentent chacun indépendamment les uns des autres un atome d'hydrogène, un groupe hydroxyle, un groupe alkyle comportant de 1 à 12 atomes de carbone ou un groupe alcoxy comportant de 1 à 12 atomes de carbone, B représente un atome de soufre ou un atome d'oxygène, et m représente 0 ou 1.

19. Sel, procédé ou composition suivant la revendication 18, où A⁺ est un contre-ion de la formule (IIIe) : 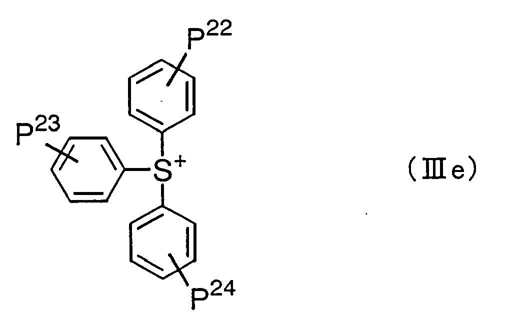 dans laquelle P²², P²³ et P²⁴ représentent chacun indépendamment les uns des autres un atome d'hydrogène ou un groupe alkyle comportant de 1 à 4 atomes.

20. Sel, procédé ou composition suivant l'une quelconque des revendications 1, 10, 11, 12, 13, 14, 15, 16, 17, 18 et 19, où le noyau X est un groupe cycloalkyle comportant de 4 à 8 atomes de carbone, un groupe adamantyle ou un groupe norbornyle, pour autant que, dans chacun des groupes, un atome d'hydrogène soit remplacé par un groupe -OH en position Q lorsque Q est un groupe -C(OH)- et que deux atomes d'hydrogène soient remplacés par =O en position Q lorsque Q est un groupe -CO-, et pour autant que, dans chacun des groupes, au moins un atome d'hydrogène puisse éventuellement être remplacé par un groupe alkyle comportant de 1 à 6 atomes de carbone, un groupe alcoxy comportant de 1 à 6 atomes de carbone, un groupe perfluoroalkyle comportant de 1 à 4 atomes de carbone, un groupe hydroxyalkyle comportant de 1 à 6 atomes de carbone, un groupe hydroxyle ou un groupe cyano.

21. Sel ou composition suivant l'une quelconque des revendications 1, 10, 11, 12, 13 et 14, où le sel est un composé de la formule (IVa) 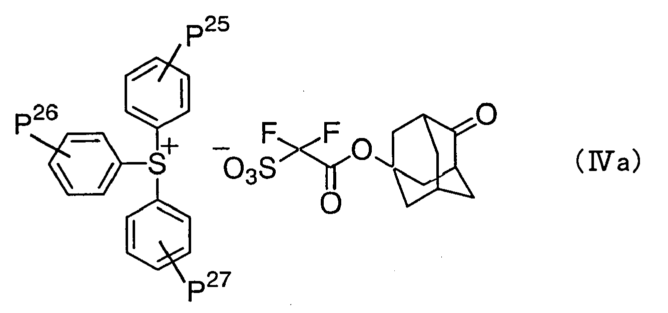 dans laquelle P²⁵, P²⁶ et P²⁷ représentent chacun indépendamment les uns des autres des atomes d'hydrogène ou un groupe alkyle comportant de 1 à 4 atomes de carbone.

22. Sel ou composition suivant l'une quelconque des revendications 1, 10, 11, 12, 13 et 14, où le sel est un composé de la formule (IVb) 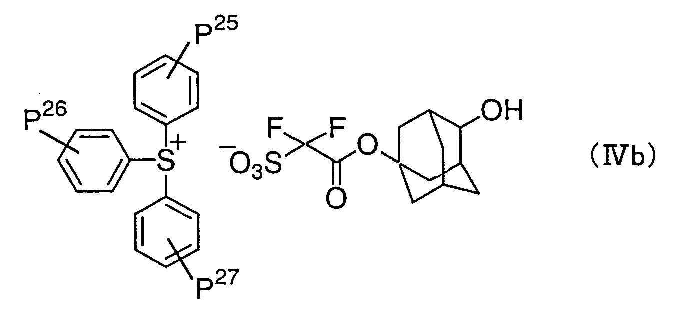 dans laquelle P²⁵, P²⁶ et P²⁷ ont les mêmes significations que celles définies dans la revendication 21.
